# EUROPEAN PATENT SPECIFICATION

(11) **EP 0 594 613 B1**
(45) Date of publication and mention of the grant of the patent: **05.11.1997**
(21) Application number: 91909813.7
(22) Date of filing: 13.05.1991
(51) Int. Cl.: G01N 33/569, C07K 7/04

(54) **SYNTHETIC PEPTIDES OF HUMAN PAPILLOMAVIRUSES 1, 6, 11, 16, 18, 31, 33 AND 56, USEFUL IN IMMUNOASSAY FOR DIAGNOSTIC PURPOSES**
SYNTHETISCHE PEPTIDE VON MENSCHLICHEN PAPILLOMAVIREN 1, 6, 11, 16, 18, 31, 33 UND 56, DIE NÜTZLICH IN IMMUNOASSAYS FÜR DIAGNOSTISCHE ZWECKE SIND
PEPTIDES SYNTHETIQUES DES VIRUS HUMAINS DU PAPILLOME NOS. 1, 6, 11, 16, 18, 31, 33, 56, UTILES A L'IMMUNOANALYS A DES FINS DIAGNOSTIQUES

(30) Priority: 11.05.1990 SE 9001705
(43) Date of publication of application: 04.05.1994
(73) Proprietor: Ferring AB, S-200 62 Malmö (SE)
(72) Inventor: DILLNER, Joakim, S-117 28 Stockholm (SE); DILLNER, Lena, S-117 28 Stockholm (SE); CHENG, Hwee-Ming, Bangsar 59000 Kuala Lumpur (MY)
(74) Representative: Ström, Tore
(86) International application number: SE9100335
(87) International publication number: WO9118294

(56) References cited:
- EP-A- 0 092 456
- EP-A- 0 256 321
- EP-A- 0 257 754
- EP-A- 0 299 354
- EP-A- 0 344 940
- EP-A- 0 375 555
- EP-A- 0 386 734
- EP-A- 0 412 762
- WO-A-87/01375
- WO-A-87/05630
- WO-A-90/04790
- DIALOG INFORMATION SERVICES, Medline, File 154; J.C. STEELE et al., AN 07256206/
- DIALOG INFORMATION SERVICES, Medline, File 154; C.A. KOMLY et al., AN 06062955/
- DIALOG INFORMATION SERVICES, Medline, File 154; C. DOORBAR et al., AN 06638652/
- PROCEEDINGS OF THE NATL. ACADEMY OF SCIENCES USA, vol. 86, May 1989, Washington, DC (US); J. DILLNER et al., p. 3838/
- DIALOG INFORMATION SERVICES, Database WPIL, File 351; TOA NENRYO KOGYO KK, AN 5195697/
- JOURNAL OF GENETIC VIROLOGY, vol. 70, 1989; D. PATEL et al., pp. 69-77/
- VIROLOGY, vol. 175, 1990; N.D. CHRISTENSEN et al., pp. 1-9/
- THE EMBO JOURNAL, vol. 6, no. 1, 1987; K. SEEDORF et al., pp. 139-144/
- INTERNATL. JOURNAL OF CANCER, vol. 45, 1990; J. DILLNER et al., pp. 529-535/
- JOURNAL OF GENETIC VIROLOGY, vol. 68, 1987; L. BANKS et al., pp. 3081-3089/
- VIROLOGY, vol. 164, 1988; J.M. FIRZLAFF et al., pp. 467-477/
- NATL. LIBRARY OF MEDLINE (NLM), Database Medline; M.S. BARBOSA et al., AN 90107938/
- NATL. LIBRARY OF MEDLINE (NLM), Database Medline; G. STRANG et al., AN 90171929/
- JOURNAL OF GENETIC VIROLOGY, vol. 67, 1986; G. MATLASHEWSKI et al., pp. 1909- 1916/
- JOURNAL OF GENETIC VIROLOGY, vol. 69, 1988; H.M. BROWNE et al., pp. 1263-1273/

## Description

The invention refers to a method for diagnosing the infection of human papillomavirus (HPV) and of papillomavirus (PV) carrying tumours, especially cervix cancer and condyloma, by the detection of virus specific antigen-antibody complexes in immunoassay. Several new immunoreactive antigens based on the HPV-types 1, 6, 11, 16, 18, 31, 33 and 56, useful for such diagnostics, have been produced by chemical means. Their use and structure is described.

Human papillomavirus is a common virus family which causes proliferative diseases in an infected epithelium. Different types of HPV cause different diseases and appear at different sites in the body. Several types, in particular type 6, 11, 16, 18, 31, 33, 35 and 52, infect the genital region. The HPV types 6 and 11 mainly cause pointed genital warts, known as condyloma acuminata. The types 16, 18, 31, 33, 35, 39, 51 and 55 cause on the other hand mainly flat, almost invisible lesions which are called cervical intraepithelial neoplasia (CIN). These CIN-lesions can develop further to cervix cancer even if comparatively seldom. More than 90 % of all cervix cancers carry some type of HPV. HPV 16 alone is found in about 60 % of all cervical tumours. HPV 1 causes benign skin tumours, verruca vulgaris or common wart. HPV 5 and HPV 8 cause malign squamous cell carcinomas in the skin, above all as a part of a state of illness with multiple warts which are called epidermodysplasia verruciformis (EV).

All HPV genoms have at least 8 regions which are supposed to code for a protein and are called open reading frames (ORF).These are numbered E1 to E7, L1 and L2. By way of introduction, the reading frames El, E2, E4, E5, E6 and E7 have been studied in connection with the present invention. Fortyone peptides in all have then been synthesized on the basis of the amino acid sequences for the proteins from E2, E4, E7, L1 or L2 of HPV 1, 5, 6, 8, 11, 16, 18, 31 and 33. The selection of peptide sequences was based on the assumption that an immunoreactive region might be situated in the same relative region of a protein from different HPV types. In this connection, the above described knowledge about the immunoreactive regions of HPV 16 and other known immunoreactive regions within E2, E4, E7, L1 and L2 of HPV 16 were utilized (J. Dillner, L. Dillner, WO 90/04790, Dillner et al., Int. J. Cancer, 45, 529-535, 1990; Dillner, Int. J. Cancer, 46, 703-711, 1990). It is not at all obvious that immunoreactive regions of the other HPV viruses should be located within the same relative regions, and as can be seen below, the successful discovery of immunoreactive regions have been very different for different peptides and different HPV types. Immunoreactive antigens of HPV 16 are known within the reading frames El, E2, and E6 (J. Dillner et al., Proceedings of the National Academy of Sciences USA, 86, 3838-3841 (1989); J. Dillner, EP,A2 344 940). Additional peptides which likewise originate from HPV 16, reading frames El, E2, E4, E6 and E7, have been described (G.K. Schoolnik, EP,A2 257 754). Moreover, these have been selected with respect to predicted secondary structure and hydrophilicity. However, these antigens differ from those claimed here.

Schoolnik et al. (EP, A2 257 754) have used a computer algorithm based on hydrophilicity and predicted secondary structure to select peptide sequences for synthesis. W. D. Lancaster presented (in a lecture at the 7th International Papillomavirus meeting, Sophia Antopolis, France, May 1988) the use of a computer algorithm to select sequences in the L1 protein of BPV-1 and BPV-2 that were either poorly conserved or exhibited homology between these BPV types. It must be emphasized that in no instance was any type of computer algorithm or other previously described methods used for peptide selection in the present invention. The reading frame E7 of HPV 16 is 84 amino acids long and has been synthesized as a 84 amino acids long peptide (Reeves et al., Lancet, i, 551-552, 1990) and as a fusion protein produced in bacteria (Jochmus-Kudielka et al., J. Nat. Cancer Inst., 81, 1698-1704, 1989). The costs for producing such long peptides are, however, very high and it is in addition technicallly difficult to accomplish and the shorter E7 peptides described here are consequently a considerable improvement.

In several investigations whole virions, whole proteins or fusion proteins of whole proteins of different HPV open reading frames have been studied (Steele et al., Virology 174, 388-398, 1990; Komly et al., J Virol. 60, 813-816, 1986; EP-B-0 092 456, WO-A-8 701 375; WO-A-8 705 630; EP-A-0 256 321; Seedorf et al., EMBO J. 6, 139-144, 1987; Banks et al., J. Gen. Virol. 68, 3061-3089, 1987; Firzlaff et al., Virology 164, 467-477, 1988). In a few instances defined epitopes as synthetic peptides have been used (EP-A-0 257 754; WO-A-9 004 790; EP-A-0 386 734; EP-A-0 275 555) though not the same peptides as in this application and with limited success in immunoassays.

An object of the invention is to provide a method of detecting especially cervix cancer in a cheaper and more easy way. Furthermore, it was intended to find peptides based on ORF E7, which should be more immunoreactive and at the same time considerbly shorter and thus cheaper and more easy to produce than the earlier described 84 amino acid long peptides. The object has been further developed to find synthetic peptides based on other medically important papillomaviruses than the earlier studied HPV 16. In the present invention we describe immunoreactive peptides from HPV 1, 6, 11, 16, 18, 31, 33 and 56.

In order to achieve said purpose the method of the invention has obtained the characterizing features of claim 1.

The invention will now be further explained below with reference to the accompanying drawings, in which
FIG 1 shows the IgA reactivity of 30 sera from patients with cervix cancer against 42 overlapping 20 amino acid peptides corresponding to the total sequence of human papillomavirus type 16 (HPV 16), ORF E1,
FIG 2 shows the IgG reactivity of 30 sera from patients with cervix cancer against 42 overlapping 20 amino acid peptides corresponding to the total sequence of human papillomavirus type 16 (HPV 16), ORF E1,
FIG 3 shows the IgM reactivity of 30 sera from patients with cervix cancer against 42 overlapping 20 amino acid peptides corresponding to the total sequence of human papillomavirus type 16 (HPV 16), ORF E1,
FIG 4 shows the IgA reactivity of 30 sera from patients with cervix cancer against 24 overlapping 20 amino acid peptides corresponding to the total sequence of human papillomavirus type 16 (HPV 16), ORF E2,
FIG 5 shows the IgG reactivity of 30 sera from patients with cervix cancer against 24 overlapping 20 amino acid peptides corresponding to the total sequence of human papillomavirus type 16 (HPV 16), ORF E2,
FIG 6 shows the IgM reactivity of 30 sera from patients with cervix cancer against 24 overlapping 20 amino acid peptides corresponding to the total sequence of human papillomavirus type 16 (HPV 16), ORF E2,
FIG 7 shows the IgA reactivity of 30 sera from patients with cervix cancer against 6 overlapping 20 amino acids peptides corresponding to the total sequence of human papillomavirus type 16 (HPV 16), ORF E4,
FIG 8 shows the IgG reactivity of 30 sera from patients with cervix cancer against 6 overlapping 20 amino acids peptides corresponding to the total sequence of human papillomavirus type 16 (HPV 16), ORF E4,
FIG 9 shows the IgM reactivity of 30 sera from patients with cervix cancer against 6 overlapping 20 amino acids peptides corresponding to the total sequence of human papillomavirus type 16 (HPV 16), ORF E4,
FIG 10 shows the IgA reactivity of 30 sera from patients with cervix cancer against 5 overlapping 20 amino acids peptides corresponding to the total sequence of human papillomavirus type 16 (HPV 16), ORF E5,
FIG 11 shows the IgG reactivity of 30 sera from patients with cervix cancer against 5 overlapping 20 amino acids peptides corresponding to the total sequence of human papillomavirus type 16 (HPV 16), ORF E5,
FIG 12 shows the IgM reactivity of 30 sera from patients with cervix cancer against 5 overlapping 20 amino acids peptides corresponding to the total sequence of human papillomavirus type 16 (HPV 16), ORF E5,
FIG 13 shows the IgA reactivity of 30 sera from patients with cervix cancer against 10 overlapping 20 amino acid peptides corresponding to the total sequence of human papillomavirus type 16 (HPV 16), ORF E6,
FIG 14 shows the IgG reactivity of 30 sera from patients with cervix cancer against 10 overlapping 20 amino acid peptides corresponding to the total sequence of human papillomavirus type 16 (HPV 16), ORF E6,
FIG 15 shows the IgM reactivity of 30 sera from patients with cervix cancer against 10 overlapping 20 amino acid peptides corresponding to the total sequence of human papillomavirus type 16 (HPV 16), ORF E6,
FIG 16 shows the IgA reactivity of 30 sera from patients with cervix cancer against 6 overlapping 20 amino acids peptides corresponding to the total sequence of human papillomavirus type 16 (HPV 16), ORF E7,
FIG 17 shows the IgG reactivity of 30 sera from patients with cervix cancer against 6 overlapping 20 amino acids peptides corresponding to the total sequence of human papillomavirus type 16 (HPV 16), ORF E7,
FIG 18 shows the IgM reactivity of 30 sera from patients with cervix cancer against 6 overlapping 20 amino acids peptides corresponding to the total sequence of human papillomavirus type 16 (HPV 16), ORF E7,
FIG 19 shows a comparison between the immunoreactivities of 30 sera from patients with cervix cancer and 60 sera from healthy persons against 4 of the examined peptides,
FIG 20 shows the mapping of papillomavirus group-specific epitopes, and
FIG 21 shows the generation of group specific antisera by immunization with synthetic peptides.

### Peptide synthesis

The polypeptides were produced synthetically and the the following formula are stated for denoting the amino acids in the synthetic peptides.

In Table 1 below the symbols indicate amin acids according to the following:

| SYMBOL | AMINO ACID |
|---|---|
| Y | L-tyrosine |
| G | glycine |
| F | L-phenylalanine |
| M | L-methionine |
| A | L-alanine |
| S | L-serine |
| I | L-isoleucine |
| L | L-leucine |
| T | L-threonine |
| V | L-valine |
| P | L-proline |
| K | L-lysine |
| H | L-histidine |
| Q | L-glutamine |
| E | L-glutamic acid |
| W | L-tryptophan |
| R | L-arginine |
| D | L-aspartic acid |
| N | L-asparagine |
| C | L-cysteine |

The different amino acids used for peptide synthesis had beeen deduced from the nucelotide sequences of the open reading frames L1, L2, E2, E4 and E7 of the HPV types 1, 6, 11, 16, 18, 31 and 33. The peptide synthesis was performed with t-Boc amino acids (Bachyem, Bubendorf, Schweiz) and a p-methylbenzhydrylamine resin (Fluka, Buchs, Schweiz) according to an earlier published method (Houghten, Proceedings of the National Academy of Sciences USA, 82, 5131-5153 1985). The protecting groups of formyltryptophan and methionine sulphoxide were removed by treatment with 25 % hydrofluoric acid and the peptides were then detached from the resin by liquid hydrofluoric acid. Since all peptides were synthesized on a p-methylbenzhydrylamine resin, all peptides contain an amide group on their carboxyterminal end.

### Preparation of peptide antisera

The peptides were coupled to the carrier protein KLH (Keyhole limpet hemocyanin, an oxygen transport protein from a marine gastropod, Sigma, St Louis, MO. USA) by using maleimidobenzoyl-n-hydroxysuccinimide ester (MBS) for peptides containing cysteine or glutardialdehyde for the others. In this connection, 4 mg peptide was coupled to 4 mg KLH. The reaction mixture with KLH was dialyzed against 10 mM phosphate buffer, pH 6, and 85 µl of 4 mg/ml MBS dissolved in dimethylformamide was then added and was allowed to react for 30 minutes. KLH-MBS was then separated from free MBS by gel filtation on a column of Sepahdex G-25 (Pharmacia, Upsala, Sweden) and the synthetic peptide was added and allowed to react for 15 hours at room temperature. When coupling with glutardialdehyde, KLH was dialyzed against PBS and 4 mg KLH was then mixed with 4 mg synthetic peptide. 200 µl of 25 % glutardialdehyd (Merck, Darmstadt, Germany) was added to 13 ml PBS. Then 260 µl of this diluted glutardialdehyde solution was added to the mixture of peptide and KLH and the reaction was allowed to proceed for 15 hours at room temperature. Finally, 100 mM Tris-HCl, pH 7.5, was added. When guinea pigs were immunized, 100 µg of the coupled peptide was injected subcutaneously. At the first immunization the peptide was suspended in 1 ml of Freunds complete adjuvant (Difco). Two weeks later and after another two weeks the animals were given 100 µg of coupled peptide in Freunds incomplete adjuvant (Difco) was administered. The animals were bled two weeks after the last immunization .

### Sera

Sera used for immunoassays were obtained from patients with HPV 16-carrying cervix cancer and from healthy controls.

### Collection of cervical secretions

A small brush (Cytobrush, Medscand, Malmö, Sweden) was rotated over the endo and exocervix and then the brush was placed in a small vial with 1 ml of phosphate buffered saline containing 5 mM ethylenedinitrilotetraacetic acid, penicilline, streptomycine and amphotericin B. The vial was mixed on a Vortex mixer and centrifuged at 5000 rpm for 10 minutes. The brush was removed and the supernatant, containing cervical secretions, was aspirated. The secretions obtained by this method were diluted 1:2 in 10 % lamb serum/phosphate buffered saline before they were used in ELISA.

### IMMUNOASSAYS

### ELISA

The synthetic peptides were diluted to 20 µg/ml in 10 mM carbonate buffer, pH 9.6, and kept for 15 hours at room temperature or, alternatively, for 72 hours at +4 °C in 50 µl/hole ELISA plates (Costar, Cambridge, Mass. USA). After a washing with phosphate buffered saline (PBS) containing 0.05 % Tween 20 (PBS-T), the plates were blocked with 10 % lamb serum in PBS (LS-PBS) for 60 minutes at 37 °C or, alternatively, for 4 hours at room temperature. Human sera were diluted 1:30 or, alternatively, 1:20 in LS-PBS, and added to the plate whereupon the mixture was incubated for 120 minuters at 37 °C. After 5 washes with PBS-T, a monoclonal antibody against IgA, labelled with peroxidase (Janssen, Beerse, Belgien) and diluted 1:500 or, alternatively, 1:200 in LS-PBS, was added. After 5 washes with PBS-T, 0.4 mg/ml of 2,2,-azino-di(3-ethylbenzthiazolinesulfonate) diammonium salt (ABTS) in 0,1 M citrate buffer, pH 4, containing 0.9 % hydrogen peroxide was added and the plate was incubated for 60 minutes. The absorbance of the liberated colour from the reaction was recorded at 415 nm in a spectrophotometer (Titertek, Flow). After 2 washings with PBS-T and blocking with LS-PBS as described above, a rabbit antibody against human IgG, labelled with alkaline phosphatase (Dako, Copenhagen, Denmark) and diluted 1:1000 in LS-PBS, was added and the mixture was incubated for 120 minutes at 37 °C. After 5 washings with PBS-T, 1 mg/ml of para-nitrophenylphosphate in 0.1 M diethanolamine buffer, pH 9,6, containing 1 mM MgCl₂ was added and the mixture was incubated for 90 minutes at room temperature. The absorbance of the liberated colour from the reaction was recorded at 405 nm in a spectrophotometer (Titertek, Flow). The plates were then washed twice with PBS-T, blocked with LS-PBS as described above and a goat antibody against human IgM, labelled with glucose oxidase (Sera-lab, England) and diluted 1:800 in LS-PBS, was then added and the mixture was incubated for 120 minutes at 37 °C. After 5 washings with PBS-T, 0.36 mg/ml of ABTS, 2.4 % glukos and 8 µg/ml peroxidase in 0.1 M phosphate buffer, pH 6.0, was added. The absorbance of the liberated colour from the reaction was recorded after 60 minutes at 405 nm in a spectrophotometer (Titertek, Flow). In every test 30 sera were allowed to react with the earlier described reactive peptide HKSAIVTLTPDSEWQRDQC as an internal standard and the absorbances were adjusted compared to the internal standard in order to eliminate variations in the results between different experiments.

### Immunofluorescence

Cells of an established cell line of mouse fibroblasts, which had been transfected with HPV type 16, and, as a control, cells of the same cell line, which did not harbour HPV 16 (NIH 3t3), were allowed to grow on a glass slide and were then fixed in 50 % acetone/50 % methanol at -20 °C for 10 minutes. After blocking of the slides with 10 % goat serum in PBS (GS-PBS) for one hour, guinea pig antisera against the different peptides, diluted 1:4 in GS-PBS, were added and the mixture was incubated for 15 hours at room temperature. The slides were immersed 60 times in PBS, 15 µg/ml of goat anti-guinea pig IgG, labelled with biotin (Vector, Burlingame,Ca. USA) in GS-PBS, was added and the mixture was incubated for 45 minutes at room temperature. After 60 immersions in PBS, Avidin labelled with fluorescein isothiocyanate (Dako, Köpenhamn, Danmark) at a concentration of 10 µg/ml in a solution of 300 mM NaCl and 10 mM Tris-HCl was added and the mixture was incubated for 30 minutes at room temperature. After 60 immersions in PBS, the specimens were mounted in 50 % glycerol and the fluorescent reactivity was determined in a fluorescence microscope (Leitz, Wetzlar, Tyskland).

### Immunohistocytochemistry

Four mikron thick sections of formalin fixated and paraffin imbedded preparations of cervix tissue, infected with HPV 16, HPV 6 or HPV 11, on glass slides were immersed in xylene, absolute ethanol, 95 % ethanol, 80 % ethanol, 50 % ethanol and, finally, PBS. Peroxidase activity was extincted by an immersion for 15 minutes in 3 % hydrogen peroxide in PBS and the slides were then blocked with 10 % goat serum in PBS (GS-PBS) for one hour. Guinea pig antisera against the different peptides, diluted 1:100 and 1:200 in GS-PBS, were added and allowed to react for 15 hours at room temperature. After 60 immersions in PBS, 15 µg/ml of goat-anti guinea pig-IgG, labelled with biotin (Vector, Burlingame,Ca. USA) in GS-PBS, was added and the mixture was incubated for 45 minutes at room temperature. After 60 immersions in PBS, Avidin labelled with peroxidase (Vector, Burlingame,Ca. USA) in PBS was added and the mixture was incubated for 30 minutes. After 60 immersions in PBS, the slides were immersed for 30 minutes in a solution of 200 ml 0.1 M acetate buffer, pH 5, containing 50 mg aminoethyl carbazole, 4 ml dimethyl formamide and 80 µl 30 % hydrogen peroxide. After 20 immersions in PBS the slides were immersed for 10 seconds in Mayer's haematoxylin and then again immersed 60 times in PBS. The specimens were then mounted with 50 % glycerol and examined under a light microscope (Leitz, Wetzlar, Germany).

### Results

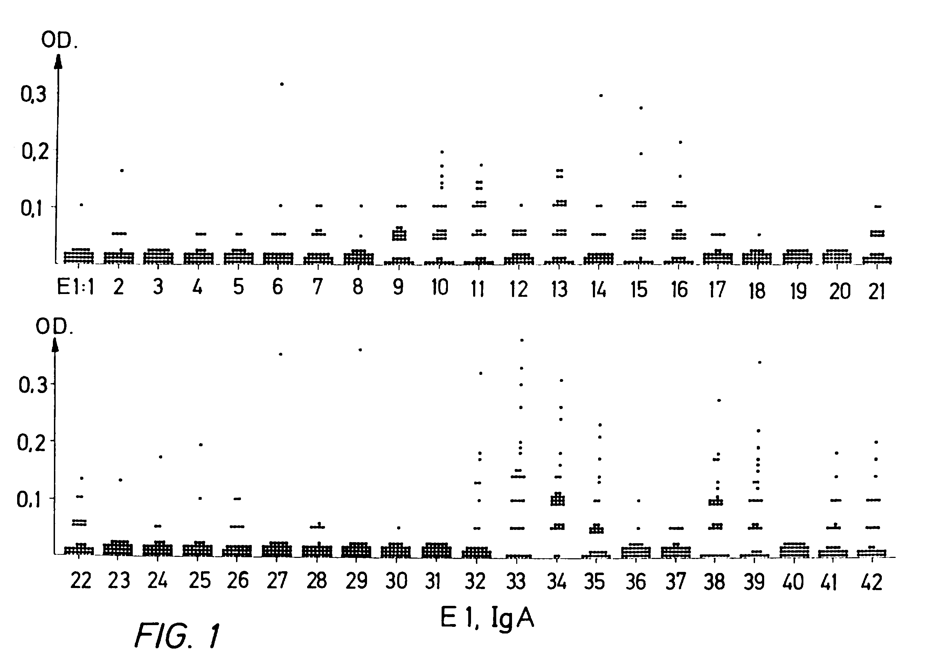

All peptides were tested in ELISA for reactivity with IgA, IgG or IgM antibodies in human sera. Peptides from HPV 16, reading frame E1, E2, E4, E5, E6 and E7 were all tested against a panel of 30 sera from patients with HPV-carrying cervix cancer. IgA reactivity against El peptides could first of all be detected with peptides from the carboxyterminal part of E1, FIG 1. The reactivity was comparatively low with the peptide E1:33 as the only peptide which was reactive with a majority of the sera.

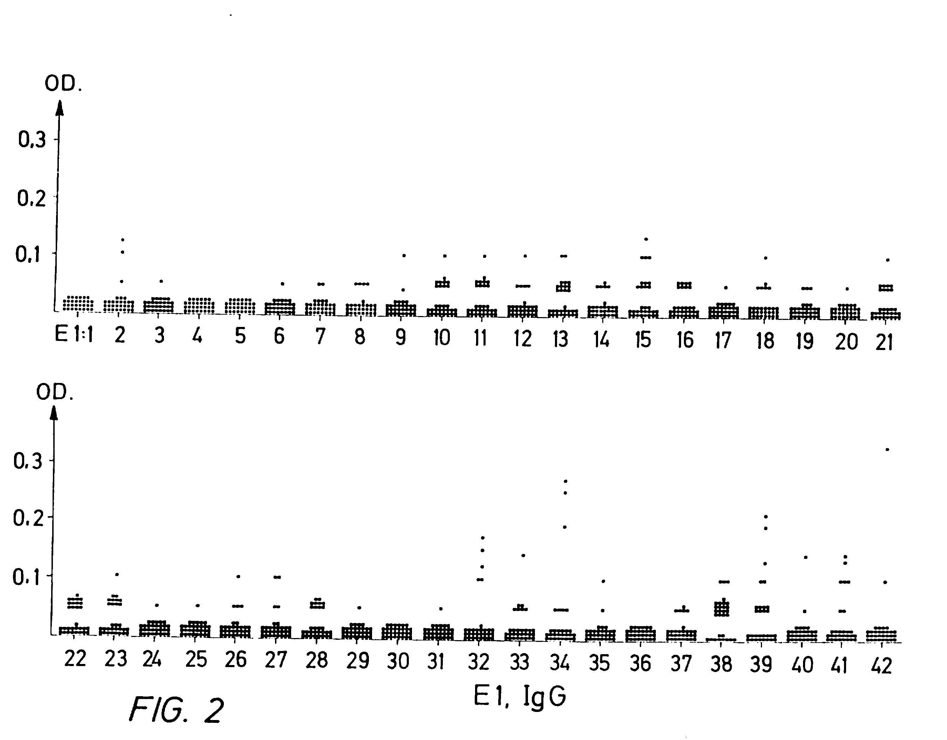

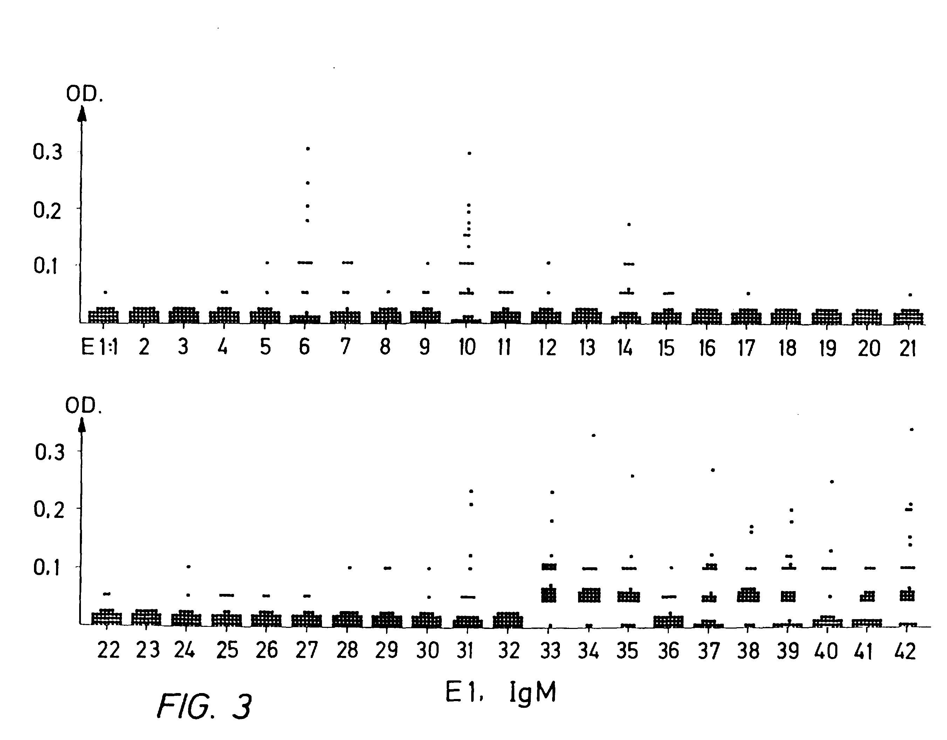

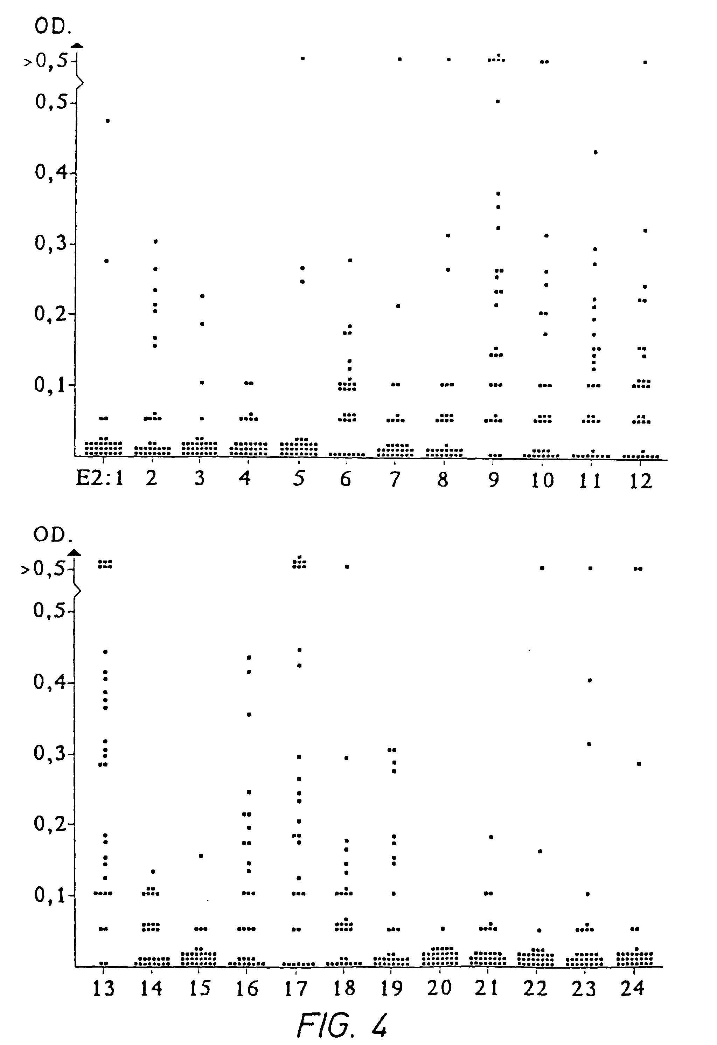

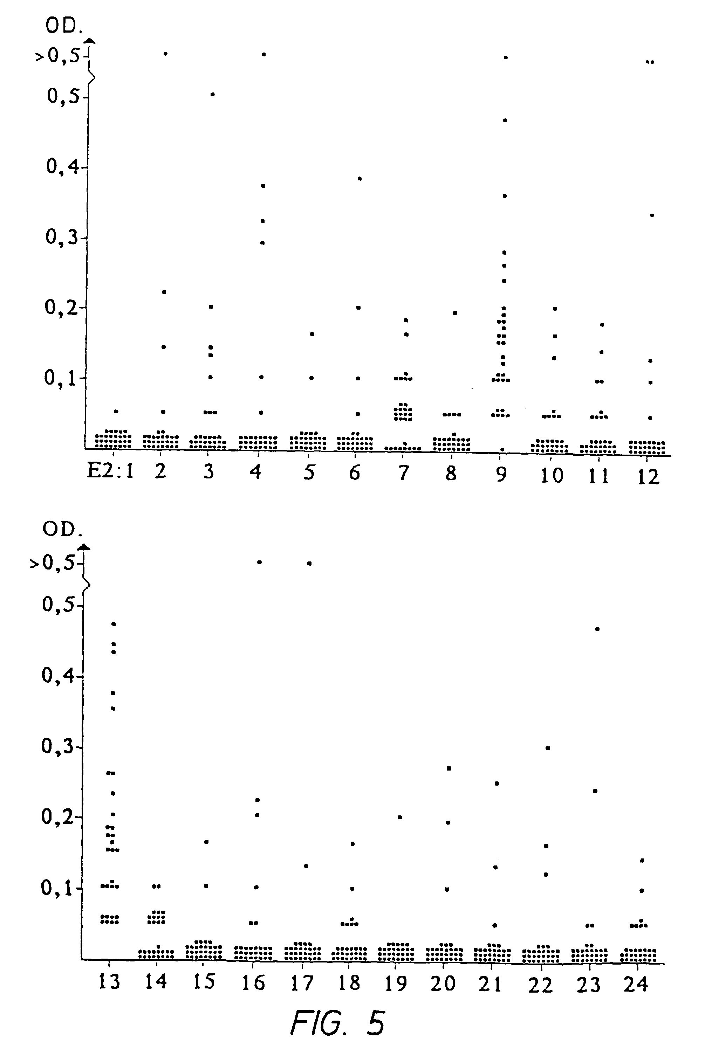

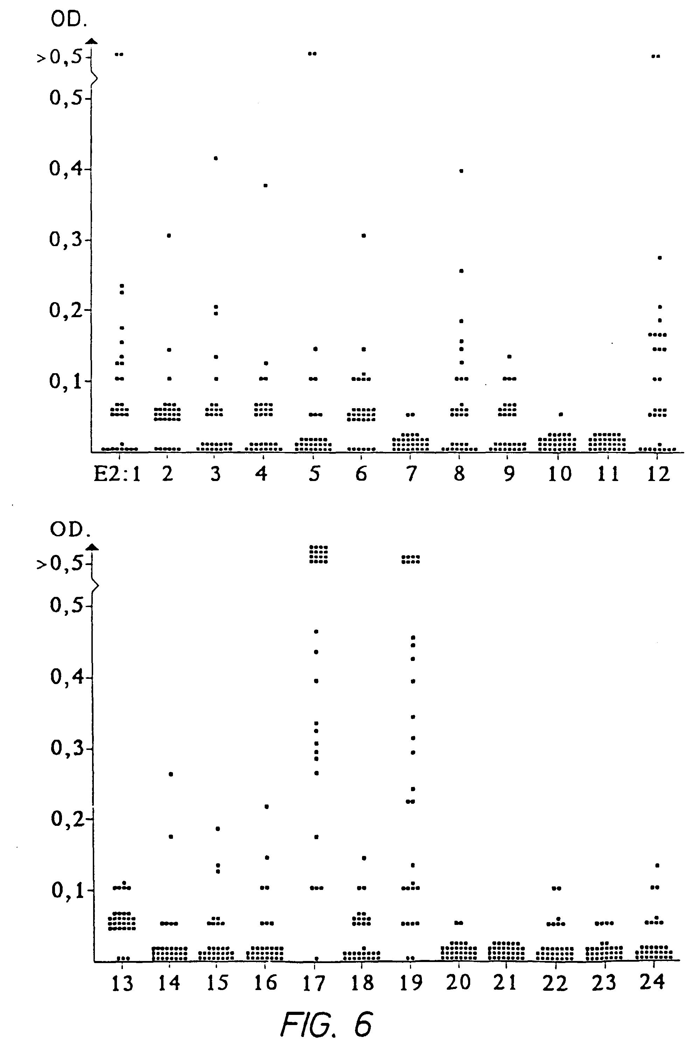

Only low IgG reactivity was obtained with a minority of the sera, FIG 2. Even the most IgG reactive peptide, E1:39, did only react with 20 % of the sera. Some IgM reactivity was also found with peptides in the carboxyterminal region of El, FIG 3, and two peptides, E1:6 and E1:10, in the aminoterminal region of El. The reading frame E2 was the most reactive of all HPV reading frames. 11 out of 24 peptides were IgA reactive with 25 % or more of the sera from patients with cervix cancer. The three most reactive peptides, E2:9, E2:13 and E2:17, were IgA reactive with 70 % or more of the sera from patients with cervix cancer, FIG 4. The IgG reactivity was somewhat lesser but two peptides, E2:9 and E2:13, were reactive with as much as 77 and 73 %, respectively, of the cervix cancer sera, FIG 5. One of the peptides which were very reactive with IgA, peptide E2:17, was not reactive with IgG to any appreciable extent. The IgM reactive peptides from E2 differed to a large extent from the IgA and IgG reactive peptides, FIG 6. For example, the IgA reactive peptides E2:9 and E2:13 were not appriciably IgM reactive, while the peptide E2:17 reacted with IgA and IgM but not with IgG.

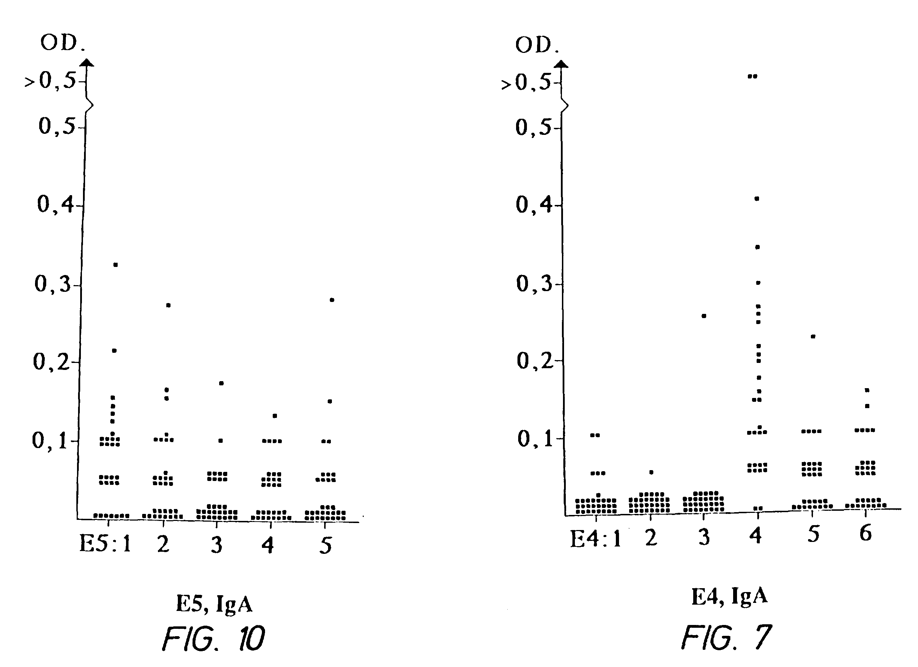

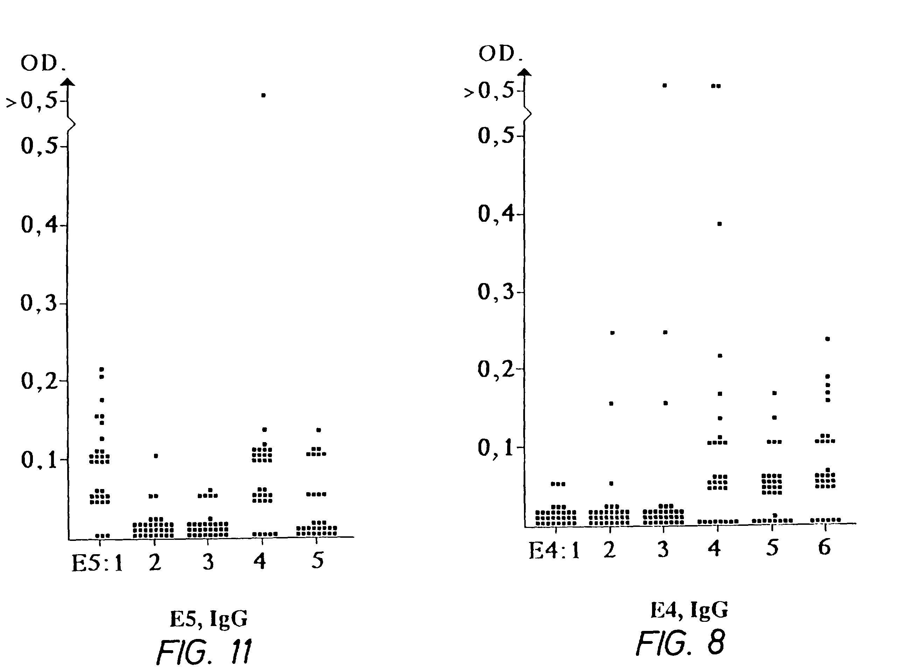

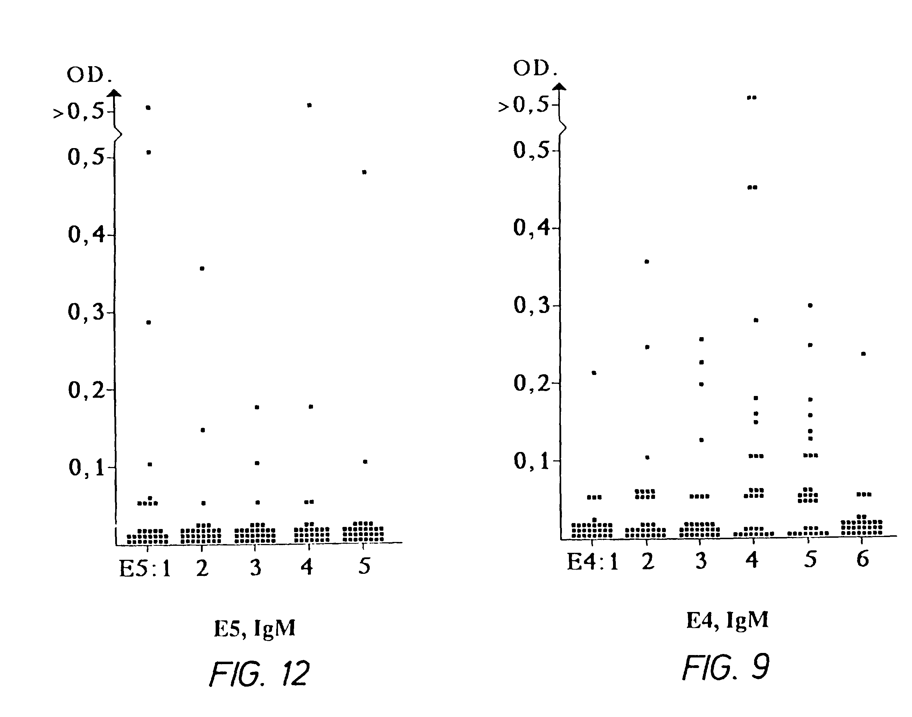

The reading frame E4 contained a main epitope for IgA antibodies, peptide E4:4, FIG 7. This peptide was also the most important E4 epitope for IgG and IgM antibodies, FIG 8 and 9. IgG reactivity was also found with peptide E4:6 at the carboxyterminal end of E4.

The reading frame E5 contained only one epitope worth mentioning, E5:1, at the aminoterminal end of E5, which had some reactivity with IgA, IgG as well as IgM, FIG 10, 11 and 12.

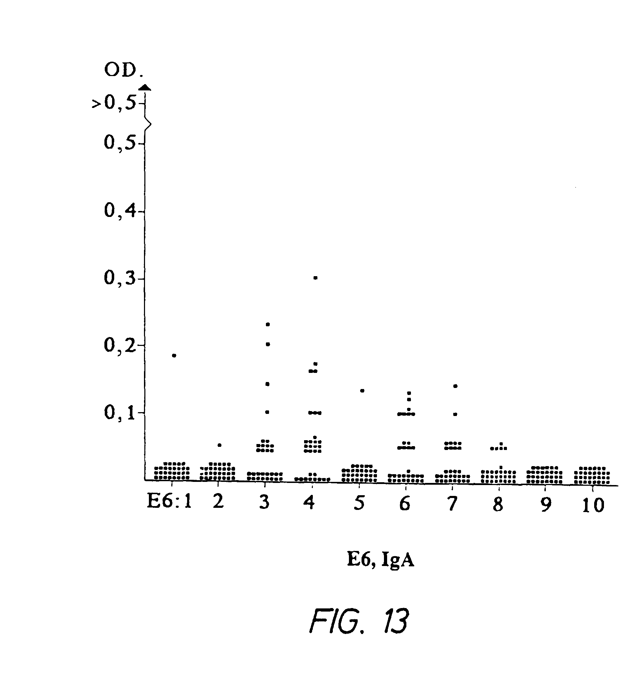

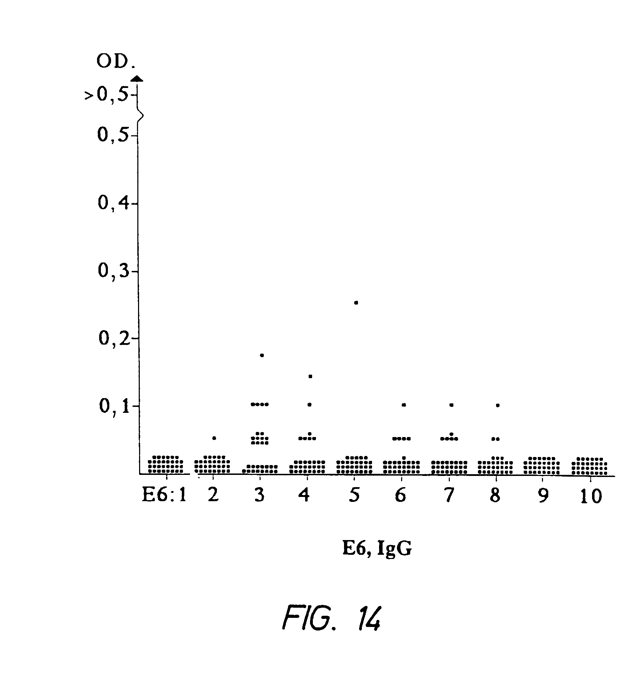

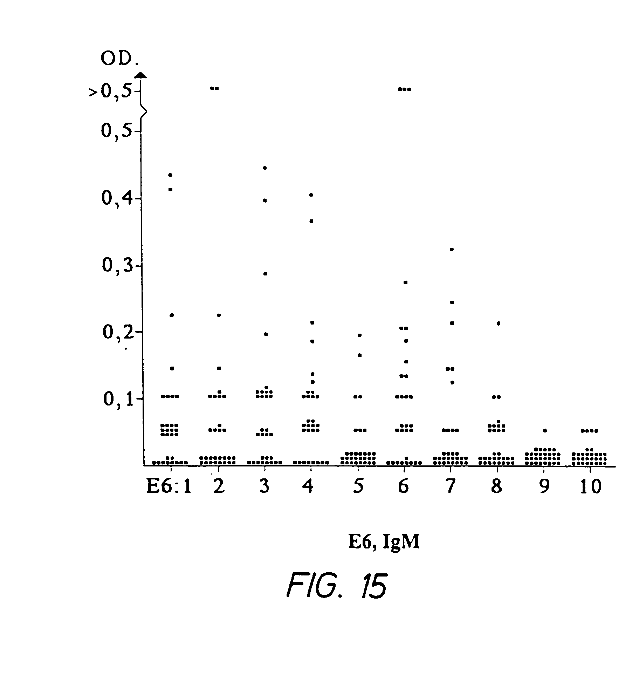

Nor was the reading frame E6 particularly imunnore-active. Comparatively low IgA and IgG reactivities were noted, FIG 13 and 14. However, several peptides were IgM reactive with the peptide E6:6 as the most reactive (47 % of the sera from patients with cervix cancer), FIG 15.

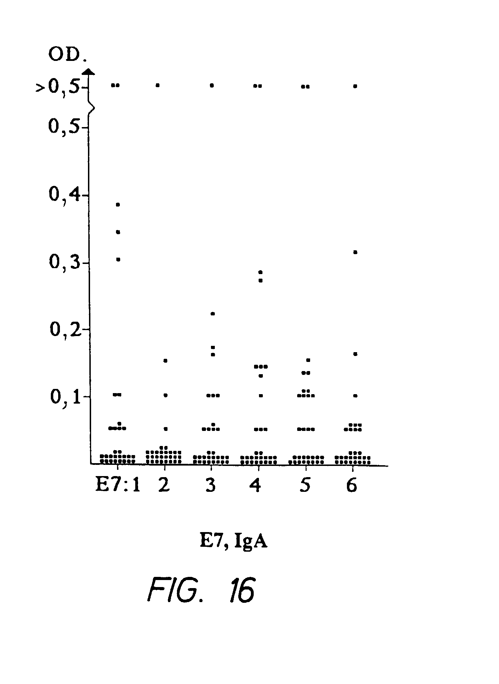

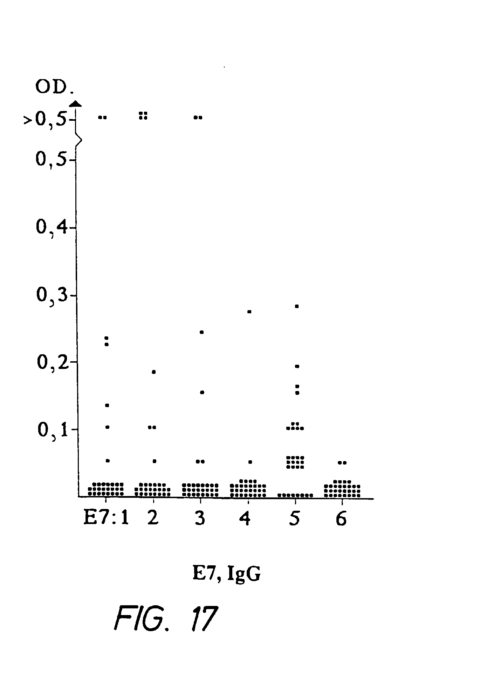

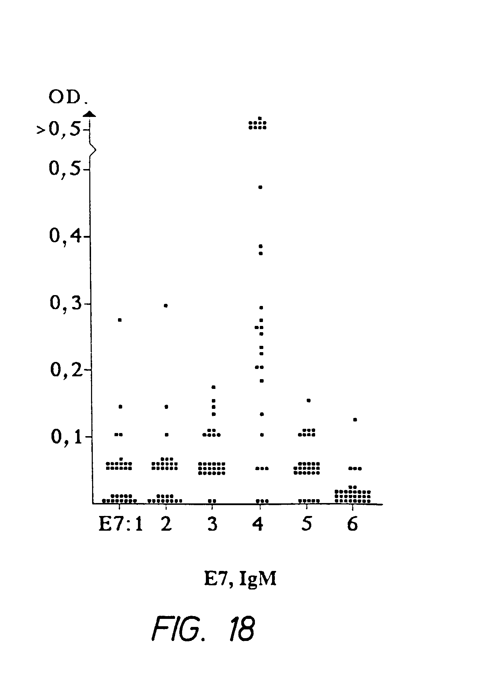

The immunoreactivity of the E7 peptides were unusual. For IgG as well as IgA, a small portion (10-20 %) of the sera exhibited extremely poternt reactivity, FIG 16 and 17. These comparatively unusual but strong reactivities were obtained for more peptides along the total E7, FIG 16 and 17. The IgM reactivity against the E7 peptides were dominated by peptide E7:4, FIG 18.

Ten of the most reactive peptides were also tested with a panel of 60 sera from healthy persons who then ought to have a smaller portion of and neither as active HPV 16 infections as the cervix cancer group. The absorbance values from ELISA for the two groups were compared with respect to statistically significant differences with aMann-Whitney test. Most of these 10 peptides were a also little reactive with the control sera as shown in Table 2 below.

**TABLE 2**

| Peptide | IgA >0.1¹ SCC | Co | p-value² | IgG >0.1 SCC | Co | p-value | IgM >0.1 SCC | Co | p-value |
|---|---|---|---|---|---|---|---|---|---|
| EI:33 | 63 % | 37 % | <0.02 | 3 % | 2 % | NS | 40 % | 20 % | NS |
| E1:39 | 43 % | 18 % | NS | 20 % | 0 % | <0.0001 | 30 % | 8 % | <0.0001 |
| E2:9 | 70 % | 62 % | <0.01 | 76 % | 82 % | NS | 13 % | 12 % | NS |
| E2:13 | 87 % | 68 % | <0.0001 | 73 % | 47 % | <0.0005 | 17 % | 20 % | NS |
| E2:17 | 70 % | 55 % | <0.01 | 7 % | 5 % | NS | 97 % | 78 % | NS |
| E2:19 | 30 % | 20 % | NS | 3 % | 2 % | NS | 80 % | 80 % | NS |
| E4:4 | 67 % | 30 % | <0.0001 | 37 % | 28 % | NS | 37 % | 37 % | NS |
| E6:6 | 23 % | 13 % | NS | 3 % | 2 % | NS | 47 % | 28 % | <0.02 |
| E7:1 | 23 % | 2 % | <0.0002 | 20 % | 8 % | <0.03 | 13 % | 2 % | NS |
| E7:4 | 30 % | 2 % | <0.0002 | 3 % | 2 % | NS | 80 % | 75 % | NS |

| | | | | | | | | | |
|---|---|---|---|---|---|---|---|---|---|
| 1) Per cent of sera which gives an absorbance value from ELISA of more than 0.1 in the group with cervical carcinoma (SCC) or in the control group (Co). | | | | | | | | | |
| 2) The numerals indicate the p-values for an significant increase in the absorbances in the group with cervical carcinoma. Notice that the p-values are not based on the percentage of positive sera but on a non-parametric method (Mann-Whitmey-test) of the actual absorbances. NS = Not significant | | | | | | | | | |

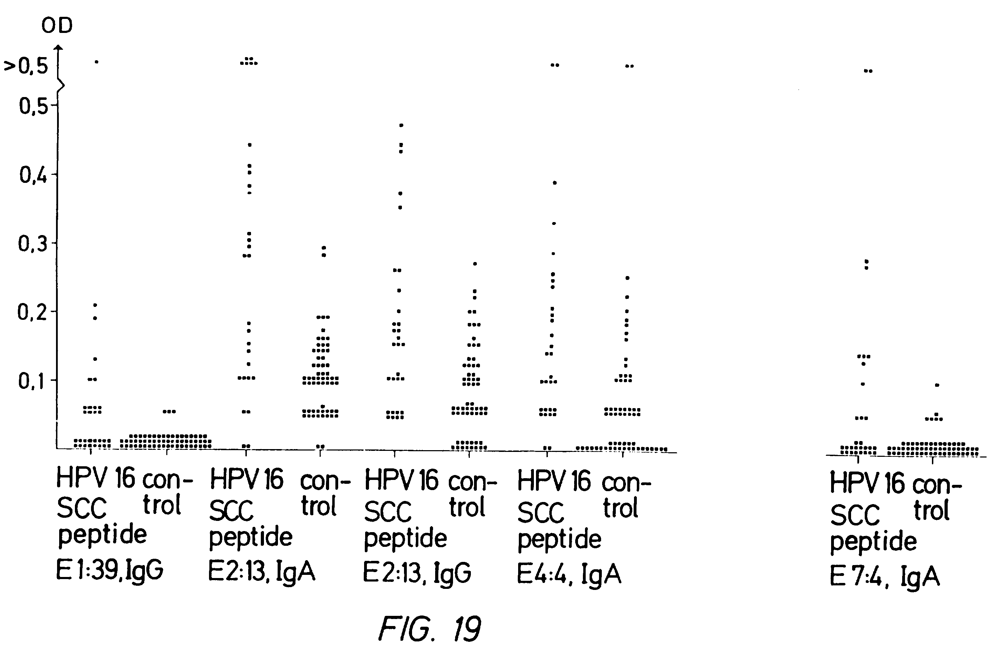

In several cases, however, very steep increases of antibody titers against these peptides were obtained with patients with cervix cancer compared with the healthy persons as is evident from Table 2 and FIG 19. The IgG reactivities against the peptides E2:13, E7:1 and E1:39 as well as the IgA reactivities against E2:9, E2:13, E2:17, E7:1, E7:4 and E4:4 should especially be mentioned. According to Table 2, the IgM reactivities were tumour associated only in a couple of cases.

Ninety-four peptides (all from E1, E2, E4, E5, E6 and E7) were also coupled to a carrier protein and used for producing anti-peptide antibodies in guinea pigs by hyperimmunization. The production of anti-peptide antibodies by hyperimmunization was verified by ELISA with a peptide which had not been coupled. All antisera were tested with respect to fluorescence against the cell lines NIH3T3 and NIH3T3/HPV 16. Several sera exhibited immunofluorescence. The antiserum against E6:1 was that serum which gave the most substantial immunofluorescence in the HPV positive cell while no fluorescence was obtained in the HPV negative cell (not shown).

All peptides were tested in ELISA with respect to reactivity with IgA, IgG or IgM antibodies in human sera. The HPV types 1, 5 and 8 infect the skin, HPV 1 gives common benign warts while HPV 5 and 8 are asssociated with malign squamous cell carcinomas in the skin, and since it is well known that warts are very frequent with children the peptides for these three types were tested with a panel of 30 sera. The first ten sera are from children (2-8 years old), the following from patients with cervix cancer, where the HPV type in the tumour has not been determined, and the last ten sera are from patients with CIN where likewise the HPV type has not been determined. The results are given as the obtained optical densities at 415 nm multiplied with 1000. Values below 100 are not considered as positive but are included for the sake of completeness.
Peptide IGSARMLVKFIDEAQREKC, HPV 1, protein E2.
   IgA: 0, 541, 918, 186, 52, 0, 48, 0, 166, 37, 5, 0, 0, 0, 0, 3, 0, 36, 51, 0, 0, 0, 0, 11, 27, 48, 17, 113, 0, 0. IgG: 185, 106, 117, 122, 183, 45, 276, 167, 94, 94, 204, 44, 80, 37, 72, 85, 176, 44, 49, 32, 85, 180, 55, 25, 51, 48, 73, 347, 40, 0.
   IgM: 102, 88, 206, 70, 87, 49, 37, 1335, 936, 151, 146, 64, 0, 39, 0, 0, 1, 0, 33, 0, 58, 33, 55, 18, 14, 46, 14, 204, 13, 28.
Peptide YDNNPDNQTRHTIWNHVYYQ, HPV 1, E2.
   IgA: 0, 28, 13, 0, 30, 3, 0, 0, 38, 9, 0, 24, 45, 0, 4, 33, 0, 84, 77, 0, 0, 16, 0, 31, 90, 114, 98, 67, 52, 0. IgG: 150, 189, 165, 144, 249, 57, 304, 206, 109, 74, 305, 98, 176, 143, 227, 172, 386, 141, 86, 107, 182, 379, 116, 92, 125, 134, 164, 271, 138, 74.
   IgM: 31, 17, 9, 105, 13, 16, 6, 311, 601, 139, 56, 93, 57, 8, 54, 38, 17, 14, 42, 24, 42, 26, 14, 23, 10, 11, 32, 17, 0, 0.

It is notable that the sera number 8 and 9 are considerably IgM positive with 3 out of 4 HPV 1 peptides.

HPV 6 and HPV 11 are two closely related viruses which cause pointed genital warts (condyloma) and rarely becomes malignant. In some rare cases can, however, HPV 6 and 11 also be associated with CIN and cervix cancer. The test panel for HPV 6 consisted of 2 sera from patients with condyloma and known HPV 6 infection, 8 sera from patients with condyloma, 4 sera from patients with cervix cancer, 6 sera from patients with condyloma, 5 sera from patients with CIN and finally further 5 sera from patients with condyloma. The test panel for HPV 11 consisted of one serum from a patient with condyloma and HPV 11, 9 sera from patients with condyloma, 4 sera from patients with cervix cancer, 6 sera from patients with condyloma, 5 sera from patients with CIN, 4 sera from patients with condyloma and finally one serum from a patient with cervix cancer.
Peptide FDGCANNTMDWVWTDVWQ, HPV 6, E2.
   IgA: 318, 0, 340, 607, 422, 242, 352, 421, 193, 112, 160, 340, 198, 171, 259, 66, 129, 101, 296, 229, 140, 206, 569, 253, 292, 119, 150, 92, 72, 236.
   IgG: 207, 229, 517, 638, 259, 430, 224, 407, 357, 257, 482, 120, 356, 333, 404, 143, 11, 276, 172, 123, 382, 674, 141, 130, 186, 304, 145, 276, 164, 241.
   IgM: 0, 0, 6, 54, 4, 1, 8, 0, 25, 0, 17, 14, 22, 5, 13, 0, 0, 5, 3, 26, 50, 62, 2, 80, 23, 2, 0, 17, 0, 20.
Peptide RLGNEHEESNSPLATPCVWP, HPV 6, E4.
   IgA: 33, 0, 61, 690, 63, 29, 99, 152, 132, 32, 0, 0, 0, 2, 80, 35, 37, 93, 56, 43, 0, 0, 0, 0, 52, 225, 48, 396, 46, 42.
   IgG:0, 0, 0, 0, 0, 0, 363, 0, 11, 0, 0, 16, 0, 0, 0, 0, 0, 7, 0, 0, 0,3, 7, 0, 0, 28, 0, 0, 0, 0.
   IgM: 0, 0, 0, 131, 0, 0, 0, 0, 18, 41, 0, 45, 49, 87, 57, 10, 0, 9, 42, 89, 135, 49, 0, 49, 0, 20, 0, 59, 126, 27.
Peptide FDGCEDNVMEYVVWTHIYLQ, HPV 11, E2.
   IgA: 178, 173, 474, 381, 229, 270, 322, 236, 122, 227, 52, 252, 170, 89, 75, 105, 181, 239, 191, 145, 190, 134, 440, 176, 151, 134, 92, 113, 169, 0.
   IgG: 140, 613, 634, 346, 314, 254, 351, 414, 207, 519, 401, 153, 383, 374, 148, 44, 368, 278, 258, 291, 387, 633, 250, 125, 177, 215, 284, 241, 251, 311.
   IgM: 0, 0, 246, 0, 0, 0, 0, 7, 0, 0, 1, 17, 8, 0, 0, 0, 0, 19, 0, 0, 13, 22, 0, 0, 0, 0, 0, 0, 0, 54.
Peptide RRRLGSEHVDRPLTTPCVWP, HPV 11, E4.
   IgA: 0, 26, 0, 0, 0, 0, 232, 22, 0, 31, 0, 0, 0, 0, 0, 0, 0, 64, 0, 41, 0, 0, 0, 5, 0, 0, 11, 0, 0, 67.
   IgG: 0, 0, 0, 0, 0, 0, 0, 0, 0, 0, 0, 0, 0, 0, 0, 0, 0, 0, 0, 0, 0, 0, 0, 0, 0, 0, 0, 0, 0, 0.
   IgM: 10, 0, 17, 0, 0, 4, 191, 0, 0, 88, 13, 45, 0, 0, 4, 0, 0, 6, 0, 0, 40, 32, 0, 0, 3, 0, 83, 0, 0, 0.
Peptide QSQAITCQKPTPEKEKQDPYK, HPV 11, L1.
   IgA: 168, 48, 143, 137, 396, 123, 282, 229, 79, 511, 61, 229, 289, 554, 247, 62, 68, 235, 69, 76, 0, 625, 0, 786, 97, 0, 49, 129, 240, 0.
   IgG: 46, 21, 122, 71, 148, 78, 46, 127, 47, 149, 150, 60, 386, 115, 78, 8, 154, 141, 100, 237, 200, 447, 115, 113, 126, 151, 204, 215, 219, 285.
   IgM: 92, 0, 139, 21, 0, 0, 0, 319, 5, 248, 134, 759, 70, 46, 11, 0, 107, 285, 141, 591, 129, 172, 254, 27, 47, 3, 205, 375, 124, 262.

HPV 16 is that HPV type which is most commonly found with cervix cancer and CIN. The test panel for HPV 16 consisted of 10 sera from patients with cervix cancer and known HPV 16 infection, 10 sera from patients with cervix cancer and finally 10 sera from patients with CIN.
Peptide HGDTPTLHEYMLDLQPETTDLYCYEQLNDS, HPV 16, E7.
   IgA: 183, 0, 0, 0, 3, 4, 80, 30, 0, 16, 18, 20, 0, 0, 0, 0, 0, 0, 34, 0, 74, 0, 188, 0, 0, 0, 12, 0, 0, 0.
   The peptide E2:9 in FIG 4, 5 and 6 had the sequence FDGDICNTMHYTNWTHIYIC. In order to see if the immunoreactivity of this peptide could be improved, we synthesized an analog of this peptide that was moved 2 amino acids compared to the original peptide in the C-terminal direction (peptide E2:9(C):
   GDICNTMHYTNWTHIYICEE). It was then tested in an IgA ELISA with a panel of 30 sera from patients with CIN (The number of IgA-positive sera in this panel were lesser than in that panel with cervical cancer sera which was used in FIG 4, 5 and 6).
Peptide E2:9:
   0, 0, 0, .105, 0, 0, 0, 0, 0, 0, 0, 0, 0, .199, 0, 0, 0, 0, 0, 0,.124, .102, .164, .126, 0, 0, 0, 0, 0, 0.
Peptide E2:9(C):
   .222, 0, .253, .136, 0, 0, .159, 0, 0, 0, 0, 0, 0, .172, 0, 0, 0,0, 0, 0, .172, .210, .233, .161, 0, 0, 0, .134, .104, .155.

As can be seen, all the sera that were positive for the original peptide were also positive for the improved peptide. In all cases but one (serum number 14, .199 versus .172) the immunoreactivity was improved when peptide E2:9(C) was used. An IgG ELISA with the same sera and peptides showed that peptide E2:9(C) was an improvement also for IgG (not shown). The peptide analog E2:9 (N) gave similar results as did peptide E2:9 (not shown).

Group-specific detection does not necessarily mean that every single virus within the papillomavirus group should be detectable but the detection should be broadly reactive with genetically diverse papillomavirus types from several host species.

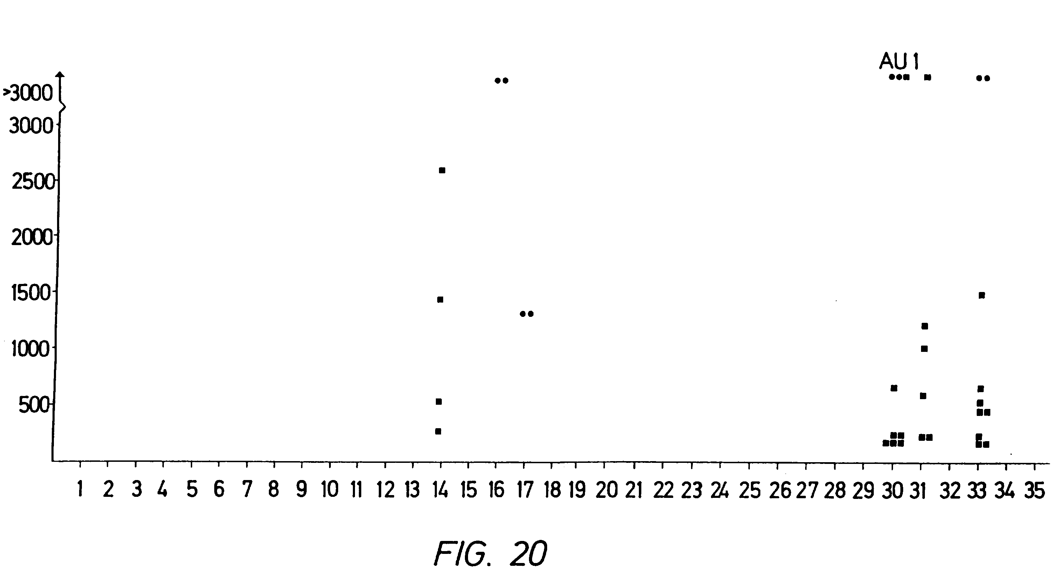

Thus, which ones of these HPV 16-derived peptides that were reactive with antibodies against bovine, canine and avian papillomaviruses was now tested to see which epitopes that were shared between these papillomaviruses with a very low degree of relatedness. As shown in FIG 20, peptides 16, 30, 31 and 33 from WO 90/04790 represented the main group-specific epitopes.

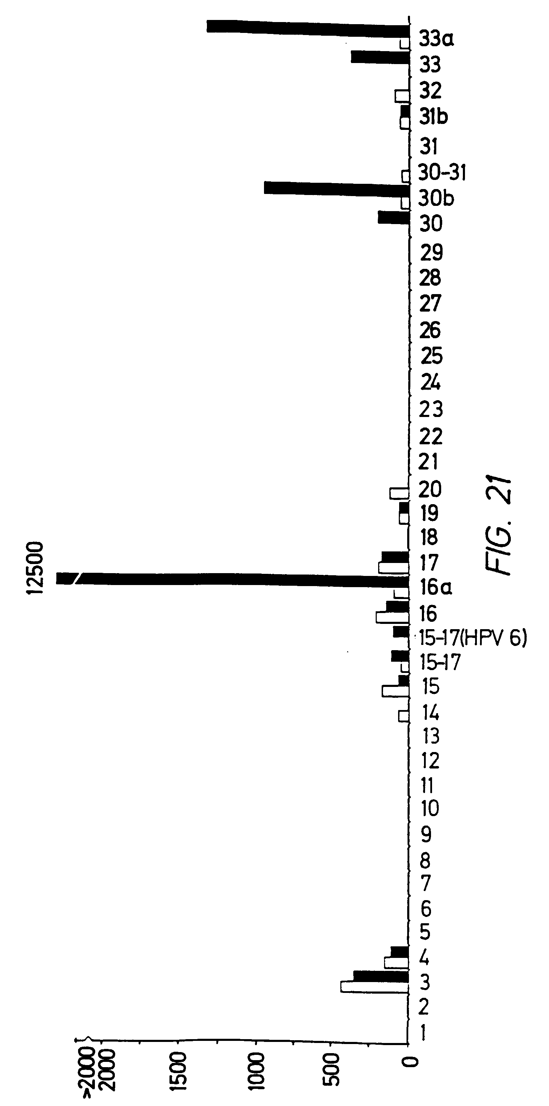

The prospects of producing antibodies against bovine papillomavirus (BPV) were then examined by immunization of guinea pigs with peptides 16, 30, 31 and 33. For comparison, antisera against all the other peptides were also produced. However, as shown in FIG 21, very low or no reactivity at all against BPV was found in ELISA.

HPV 18 is a common virus associated with CIN and cervix cancer, especially adenocarcinoma. The test panel for HPV 18 consisted of 7 sera from patients with cervix cancer and known HPV 18 infection, 2 sera from patients with CIN and known HPV 18 infection, 11 sera from patients with cervix cancer and finally 10 sera from patients with CIN.
Peptide FDGNKDNCMTYVAWDSVYYM, HPV 18, E2.
   IgA: 27, 3, 0, 22, 15, 89, 74, 75, 7, 0, 0, 60, 60, 0, 0, 19, 19, 24, 83, 0, 84, 0, 393, 79, 98, 146, 0, 36, 0.
   IgG: 282, 371, 222, 146, 220, 315, 442, 339, 169, 0, 581, 176, 406, 412, 498, 300, 696, 306, 192, 194, 438, 628, 279, 157, 329, 280, 233, 357, 162, 75.
   IgM: 42, 55, 0, 28, 39, 30, 28, 0, 0, 0, 85, 49, 103, 25, 48, 28, 31, 18, 41, 31, 76, 76, 50, 30, 46, 22, 66, 21, 29, 0.
Peptide SLLNSYSTPPHRIPAPCPWA, HPV 18, E4.
   IgA: 34, 0, 0, 0, 61, 46, 62, 0, 0, 0, 0, 17, 35, 0, 5, 0, 0, 0, 35, 0, 0, 0, 293, 0, 17, 0, 14, 32, 0, 0.
   IgG: 0, 0, 0, 3, 0, 0, 0, 0, 0, 0, 0, 33, 0, 0, 0, 0, 0, 0, 0, 0, 0, 0, 0, 9, 0, 0, 0.
   IgM: 24, 0, 0, 0, 10, 9, 0, 0, 1, 0, 0, 17, 36, 2, 4, 0, 20, 12, 30, 0, 33, 78, 0, 0, 62, 0, 0, 31, 0, 0.
Peptide HGPKATLQDIVLHLEPQNEIPVDLLCHEQL, HPV 18, E7.
   IgA: 171, 48, 0, 21, 77, 108, 294, 119, 24, 0, 41, 271, 80, 40, 0, 121, 181, 37, 252, 48, 148, 145, 517, 156, 150, 212, 236, 17, 155, 0.
   IgG: 0, 17, 0, 21, 4, 4, 4, 0, 0, 0, 23, 9, 14, 0, 0, 3, 8, 0, 0, 0, 0, 38, 0, 0, 0, 0, 0, 0, 0, 0.
   IgM: 17, 38, 0, 22, 22, 31, 10, 0, 1, 0, 41, 65, 12, 8, 19, 19, 0, 31, 0, 65, 38, 0, 4, 8, 18, 23, 0, 0, 0.
Peptide QHLPARRWPQRHTMLCMCCKCEARIELVV, HPV 18, E7.
   IgA: 331, 236, 83, 7, 193, 202, 141, 0, 30, 266, 144, 627, 301, 155, 14, 440, 213, 94, 662, 0, 0, 971, 0, 0, 4, 520, 243, 506, 102, 0.
   IgG: 0, 34, 0, 0, 13, 6, 0, 0, 0, 0, 14, 63, 31, 13, 8, 19, 0, 7, 7, 0, 1, 122, 0, 0, 0, 156, 16, 26, 39, 0.
   IgM: 23, 143, 59, 162, 131, 143, 29, 121, 96, 136, 82, 80, 61, 130, 104, 83, 63, 99, 124, 58, 137, 22, 22, 46, 129, 146, 131, 27, 44, 121.
Peptide LCMCCKCEARIELVVESSADDLRAFQQLFL, HPV 18, E7.
   IgA: 374, 28, 0, 44, 137, 205, 484, 27, 28, 71, 114, 1835, 23, 87, 10, 155, 103, 435, 360, 492, 145, 74, 339, 540, 243, 453, 225, 0, 219, 0.
   IgG: 565, 606, 438, 266, 414, 546, 758, 432, 275, 468, 823, 195, 492, 687, 710, 466, 929, 346, 331, 185, 663, 952, 464, 335, 381, 399, 260, 386, 211, 100.
   IgM: 26, 39, 0, 32, 40, 109, 119, 15, 66, 29, 77, 28, 0, 137, 0, 31, 20, 156, 116, 50, 135, 45, 30, 60, 68, 49, 50, 30, 92, 10.
Peptide ESSADDLRAFQQLFLNTLSFVCPWCASQQ, HPV 18, E7.
   IgA: 91, 12, 0, 7, 104, 243, 140, 0, 5, 18, 0, 87, 0, 0, 0, 60, 0, 0, 151, 0, 0, 79, 0, 15, 72, 155, 121, 0, 102, 0.
   IgG: 331, 376, 287, 226, 271, 304, 438, 217, 147, 216, 392, 186, 354, 280, 366, 319, 551, 229, 218, 135, 325, 520, 254, 181, 190, 129, 140, 239, 92, 39.
   IgM: 55, 103, 0, 81, 152, 417, 98, 36, 52, 7, 32, 205, 36, 43, 0, 27, 53, 24, 52, 22, 151, 125, 180, 76, 139, 156, 142, 36, 352, 153.
Peptide QSVAITCQKDAAPAENKDPYD, HPV 18, L1.
   IgA: 223, 0, 0, 0, 0, 45, 0, 53, 0, 0, 0, 0, 10, 0, 0, 32, 0, 0, 4, 0, 0, 0, 1, 0, 9, 0, 28, 66, 24, 0.
   IgG: 226, 233, 192, 90, 126, 597, 286, 248, 138, 0, 376, 46, 135, 83, 149, 118, 320, 108, 320, 108, 66, 117, 272, 435, 108, 30, 99, 118, 94, 405, 99, 25.
   IgM: 50, 9, 99, 58, 12, 0, 0, 20, 65, 0, 32, 57, 24, 0, 0, 0, 0, 0, 13, 0, 25, 48, 70, 12, 95, 0, 0, 42, 115, 29.

HPV 31 is also a common virus in connection with CIN and is sometimes seen in connection with cervix cancer. The test panel for HPV 31 consisted of 10 sera from patients with CIN and known HPV 31 infection, 10 sera from patients with cervix cancer and 10 sera from patients with CIN.

HPV 33 is a relatively new virus which has been found to be rather common both in connection with CIN and cervix cancer. The test panel for HPV 33 consisted of 4 sera from patients with cervix cancer and known HPV 33 infection, 5 sera from patients with CIN and known HPV 33 infection, 11 sera from patients with cervix cancer and finally 10 sera from patients with CIN.
Peptide YDNDKKNTMDYTNWGEIYII, HPV 33, E2.
   IgA: 53, 0, 29, 204, 67, 65, 19, 83, 210, 47, 12, 170, 77, 551, 35, 140, 146, 71, 150, 103, 210, 87, 347, 143, 180, 222, 241, 110, 171, 0.
   IgG: 165, 268, 2202, 237, 240, 415, 97, 211, 333, 147, 458, 191, 323, 384, 330, 250, 714, 185, 146, 142, 459, 541, 250, 110, 124, 242, 148, 335, 149, 65.
   IgM: 4, 0, 0, 0, 0, 0, 0, 0, 2, 155, 7, 0, 0, 0, 0, 0, 0, 0, 0, 0, 0, 24, 0, 0, 0, 0, 0, 7, 0, 0.
Peptide PQTPPSPLQSCSVQTPPWTI, HPV 33, E4.
   IgA: 92, 0, 28, 14, 0, 22, 7, 38, 127, 62, 73, 200, 74, 138, 44, 227, 49, 61, 283, 0, 41, 121, 69, 88, 145, 68, 37, 81, 84, 0.
   IgG: 6, 5, 0, 1, 19, 0, 0, 0, 0, 0, 0, 27, 0, 15, 0, 0, 0, 20, 0, 0, 0, 0, 0, 0, 0, 0, 0, 0, 0, 0.
   IgM: 355, 66, 163, 12, 73, 94, 32, 212, 55, 80, 136, 374, 71, 112, 57, 210, 40, 31, 650, 37, 627, 222, 45, 168, 50, 31, 37, 51, 88, 118.
Peptide TSQAITCQKTVPPKEKEDPLG, HPV 33, L1.
   IgG: 139, 128, 936, 57, 82, 82, 220, 39, 86, 127, 264, 48, 87, 34, 111, 89, 261, 50, 69, 29, 184, 316, 93, 0, 28, 21, 34, 237, 35, 0.
   IgM: 5, 0, 0, 0, 55, 78, 31, 112, 209, 122, 105, 304, 137, 14, 37, 0, 1, 9, 4, 0, 99, 150, 149, 0, 0, 11, 56, 123, 190, 108.
   E7 peptides for HPV types 1, 5, 6, 8, 11, 18, 31, 33 and 56.

An HPV 16 peptide from the E7 open reading frame which had the sequence
CCKCDSTLRLCVQSTHVDIRTLEDLLMGTL
was studied. Analysis of the predicted amino acid sequences revealed that all papillomaviruses have a motif of 30 amino acids in the E7 open reading frame that can be represented by CxxCxxxxxxxxxxxxxxxxxxxxLL'xxxL', where L' usually is Leucine, but in a few cases was substituted for the other hydrophobic amino acids valine or Phenylalanine.

The peptides described by this formula were synthesized for HPV 1, 6, 11, 18, 31, 33 and 56 and tested in IgA and IgG ELISAs with a panel of 30 sera from patients with CIN.
Peptide CCGCDSNVRLVVQCTETDIREVQQLLLGTL (HPV 6, E7),
   IgA: .162, 0, .182, .228, 0, 0, 0, 0, 0, 0, 0, 0, .122, .185, 0, 0, 0, 0, 0, 0, .134, .197, .227, .100, 0, .104, 0, .117, 0, .121.
   IgG: .240, .287, .130, 0, 0, .185, 0, 0, .224, 0, 0, .145, 0, .130, 0, 0, 0, 0, 0, 0, 0, .401, 0, 0, .161, 0, 0, 0, 0, 0, .126.
Peptide CAYCEKLVRLTVLADHSAIRQLEELLLRSL (HPV 1, E7),
   IgA: .128, 0, .142, .125, 0, 0, 0, 0, 0, 0, 0, 0,.115, .230, 0, 0, 0, 0, 0, 0, .120, .121, .162, .115, 0, 0, 0, .121, 0, 0.
   IgG: .229, .176, .108, 0, 0, 0, 0, 0, .123, 0, 0, 0, 0, .121, 0, 0, 0, 0, 0, 0, .166, 0, 0, 0, .130, 0, 0, 0, 0, .105
Peptide CHTCNTTVRLCVNSTASDLRTIQQLLMGTV (HPV 33, E7),
   IgA: 0, .178, 0, 0, 0, 0, .115, .103, .115, 0, 0, 0, .115, .339, 0, .148, .145, .105, .205, .350, .112, .130, .138, .196, 0, 0, 0, .117, 0, .132.
   IgG: .148, .301, .114, 0, .134, .110, 0, .180, .161, 0, 0, .145, 0, .170, 0, 0, 0, .130, .143, .161, 0, 0, 0, .127, .112, 0, 0, 0, 0, .111.
Peptide CCGCDSNVRLVVECTDGDIRQLQDTTTGTL (HPV 11, E7),
   IgA: .253, 0, .286, .142, 0, 0, 0, 0, 0, 0, 0, 0, 0, .230, 0, 0, 0, 0, 0, 0, .199, .229, .226, .158, 0, 0, 0, .107, 0, .123.
   IgG: .325, .464, .259, 0, 0, .293, 0, 0, .307, .133, 0, .219, 0, .178, 0, 0, 0, 0, 0, 0, .699, 0, 0, .261, 0, 0, 0, 0, 0, .210.
Peptide CCKCEARIELVVESSADDLRAFQQLFLNTL (HPV 18, E7),
   IgA: .143, 0, .292, .133, 0, .127, 0, 0, 0, 0, 0, 0, 0, .221, 0, 0, 0, 0, 0, 0, .129, .206, .200, .141, 0, 0, .123, .135, 0, 175.
   IgG: .239, .276, .200, 0, 0, .182, 0, 0, .199, 0, 0, .181, 0, 0, 0, 0, 0, 0, 0, 0, .476, .167, 0, .171, 0, 0, 0, 0, 0, 0.
Peptide CCECKFVVQLDIQSTKEDLRVVQQLLMGAL (HPV 56, E7),
   IgA: .149, 0, .171, 0, 0, 0, 0, 0, 0, 0, 0, 0, .117, .260, 0, 0, 0, .112, 0, 0, .132, .145, .135, .120, 0, 0, 0, .132, 0, .122.
   IgG: .134, .224, 0, 0, 0, .110, 0, 0, .113, 0, 0, 0, 0, .105, 0, 0, 0, 0, 0, 0, .182, 0, 0, 0, 0, 0, 0, 0, 0, 0.
Peptide CCQCKSTLRLCVQSTQVDIRILQELLMGSF (HPV 31, E7),
   IgA: 1.344, .200, .855, .600, 0, .358, .282, 0, .158, 0, 0, 0, 0, .694, .122, 0, 0, 0, 0, .107, .902, .948, 1.762, .882, 0, 0, 0, 0, .121, .451.
   IgG: .448, .775, .435, .123, 0, .530, .411, 0, .529, .405, 0, .825, 0, .538, .179, 0, 0, 0, 0, 0, .588, .198, 0, .635, 0, 0, 0, .422, .163, .474

As shown by the results presented above, this site in the E7 protein is an important antigenic site (for both IgA and IgG) that was present for all HPV types tested except HPV 5.

### Antibodies in cervical secretions

The major immunoreactive peptides were also tested in IgA and IgG ELISAs with cervical secretions from 30 women with cervical intraepithelial neoplasia (CIN) or with a history of CIN. It was found that those peptides which were the most immunoreactive with serum also were those which were most reactive with cervical secretions.

The results are exemplified with peptide E2:9 (FDGDICNTMHYTNWTHIYIC). IgA-ELISA where the results are given as optical densities for a 1:2 dilution of the secretion:
.063, 0, .059, 0, .091, .456, .074, 0, 0, 0, .177, 0, 1.563, 0,.124, 0, 0, 0, 0, 0, .104, .083, 0, 0, 0, .068, 0, 0, 0, .150.
IgG-ELISA also at a 1:2 dilution of the secretion:
.080, 0, 0, 0, .067, .262, .110, 0, 0, 0, .131, 0, .092, 0, 0, 0,0, 0, 0, 0, 0, 0, 0, 0, 0, 0, 0, 0, 0, .070.

As seen from the results shown above, IgA was the major Ig class in secretions. An ELISA was also performed on serum from the same patients. Comparison of the IgG-results for serum and secretions showed a strong correlation, whereas no obvious correlation was seen between IgA in serum and secretions (not shown). This suggests that most of the IgA found in secretions was produced locally, whereas the IgG found in secretions may be due to a leakage of serum antibodies into the secretions.

In summary, these results show that several immunoreactive peptides based on both HPV 1, 6, 11, 16, 18, 31, 33 and 56 have been found. The immunoreactivity is quite different depending on if IgG, IgA or IgM is measured. The method used here, ELISA, is only one of several variants of immunoasssay, but the most simple and most practical method for testing sera on a large scale.

## Claims

1. Method for diagnosing the presence of human papillomavirus (HPV) infection and of papillomavirus (PV) carrying tumours by immunoassay, **characterized** in that the detection is performed by immunoassay based on any of the peptides 
IGSARMLVKFIDEAQREKC (HPV 1, E2)
YDNNPDNQTRHTIWNHVYYQ (HPV 1, E2)
CAYCEKLVRLTVLADHSAIRQLEELLLRSL (HPV 1, E7)
FDGCANNTMDYVVWTDVYVQ (HPV 6, E2)
RLGNEHEESNSPLATPCVWP (HPV 6, E4)
CCGCDSNVRLVVQCTETDIREVQQLLLGTL (HPV 6, E7)
FDGCEDNVMEYVVWTHIYLQ (HPV 11, E2)
RRRLGSEHVDRPLTTPCVWP (HPV 11, E4)
CCGCDSNVRLVVECTDGDIRQLQDLLLGTL (HPV 11, E7)
QSQAITCQKPTPEKEKQDPYK (HPV 11, L1)
KQHRDAVQVLKRKYLGSCIE (HPV 16, E1)
QYSGGSGGGCSQYSSGSGGE (HPV 16, E1)
GSGGEGVSERHTICQTPLTN (HPV 16, E1)
KAAMLAKFKELYGVSFSELV (HPV 16, E1)
SKSHFWLQPLADAKIGMLDD (HPV 16, E1)
GMLDDATVPCWNYIDDNLRN (HPV 16, E1)
LHNRLVVFTFPNEFPFDENG (HPV 16, E1)
FDENGNPVYELNDKNWKS (HPV 16, E1)
GDICNTMHYTNWTHIYICEE (HPV 16, E2)
PTGCIKKHGYTVEVQFDGDI (HPV 16, E2)
FDGDICNTMHYTNWTHIYIC (HPV 16, E2)
QVDYYGLYYVHEGIRTYFVQ (HPV 16, E2)
TYFVQFKDDAEKYSKNKVWE (HPV 16, E2)
PRSEPDTGNPCHTTKLLHRD (HPV 16, E2)
TNLDTASTTLLACFLLCFCV (HPV 16, E5)
YSKISEYRHYCYSLYGTTLE (HPV 16, E6)
GTTLEQQYNKPLCDLLIRCI (HPV 16, E6)
FDGNKDNCMTYVAWDSVYYM (HPV 18, E2)
SLLNSYSTPPHRIPAPCPWA (HPV 18, E4)
 
CCKCEARIELVVESSADDLRAFQQLFLNTL (HPV 18, E7)
HGPKATLQDIVLHLEPQNEIPVDLLCHEQL (HPV 18, E7)
QHLPARRAEPQRHTMLCMCCKCEARIELVV (HPV 18, E7)
LCMCCKCEARIELVVESSADDLRAFQQLFL (HPV 18, E7)
ESSADDLRAFQQLFLNTLSFVCPWCASQQ (HPV 18, E7)
QSVAITCQKDAAPAENKDPYD (HPV 18, L1)
CCQCKSTLRLCVQSTQVDIRILQELLMGSF (HPV 31, E7)
YDNDKKNTMDYTNWGEIYII (HPV 33, E2)
PQTPPSPLQSCSVQTPPWTI (HPV 33, E4)
CHTCNTTVRLCVNSTASDLRTIQQLLMGTV (HPV 33, E7)
CCECKFVVQLDIQSTKEDLRVVQQLLMGAL (HPV 56, E7)
 or the detection of a virus specific antigen-antibody complex in the immunoassay being detected by a peptide containing the same epitope or a peptide containing substantial homology with the original peptide.

2. Method for diagnosing the presence of human papillomavirus (HPV) infection and of papillomavirus (PV) carrying tumours by immunoassay, **characterized** in that the detection is performed by immunoassay based on IgA antibodies against any of the peptides 
CAYCEKLVRLTVLADHSAIRQLEELLLRSL  (HPV 1, E7)
FDGCANNTMDYVVWTDVYVQ  (HPV 6, E2)
CCGCDSNVRLVVQCTETDIREVQQLLLGTL  (HPV 6, E7)
FDGCEDNVMEYVVWTHIYLQ  (HPV 11, E2)
CCGCDSNVRLVVECTDGDIRQLQDLLLGTL  (HPV 11, E7)
QYSGGSGGGCSQYSSGSGGE  (HPV 16, E1)
GSGGEGVSERHTICQTPLTN  (HPV 16, E1)
KAAMLAKFKELYGVSFSELV  (HPV 16, E1)
SKSHFWLQPLADAKIGMLDD  (HPV 16, E1)
GMLDDATVPCWNYIDDNLRN  (HPV 16, E1)
LHNRLVVFTFPNEFPFDENG  (HPV 16, E1)
FDENGNPVYELNDKNWKS  (HPV 16, E1)
GDICNTMHYTNWTHIYICEE  (HPV 16, E2)
FDGDICNTMHYTNWTHIYIC  (HPV 16, E2)
QVDYYGLYYVHEGIRTYFVQ  (HPV 16, E2)
 
TYFVQFKDDAEKYSKNKVWE (HPV 16, E2)
PRSEPDTGNPCHTTKLLHRD (HPV 16, E2)
TNLDTASTTLLACFLLCFCV (HPV 16, E5)
FDGNKDNCMTYVAWDSVYYM (HPV 18, E2)
CCKCEARIELVVESSADDLRAFQQLFLNTL (HPV 18, E7)
CCQCKSTLRLCVQSTQVDIRILQELLMGSF (HPV 31, E7)
CHTCNTTVRLCVNSTASDLRTIQQLLMGTV (HPV 33, E7)
CCECKFVVQLDIQSTKEDLRVVQQLLMGAL (HPV 56, E7)
 or the detection of a virus specific antigen-antibody complex in the immunoassay being detected by a peptide containing the same epitope or a peptide containing substantial homology with the original peptide.

3. Method for diagnosing the presence of human papillomavirus (HPV) infection and of papillomavirus (PV) carrying tumours by immunoassay, **characterized** in that the detection is performed by immunoassay based on IgG antibodies against any of the peptides 
IGSARMLVKFIDEAQREKC  (HPV1, E2)
YDNNPDNQTRHTIWNHVYYQ  (HPV 1, E2)
CAYCEKLVRLTVLADHSAIRQLEELLLRSL  (HPV 1, E7)
FDGCANNTMDYVVWTDVYVQ  (HPV 6, E2)
RLGNEHEESNSPLATPCVWP  (HPV 6, E4)
CCGCDSNVRLVVQCTETDIREVQQLLLGTL  (HPV 6, E7)
FDGCEDNVMEYVVWTHIYLQ  (HPV 11, E2)
CCGCDSNVRLVVECTDGDIRQLQDLLLGTL  (HPV 11, E7)
QSQAITCQKPTPEKEKQDPYK  (HPV 11, L1)
GMLDDATVPCWNYIDDNLRN  (HPV 16, E1)
FDENGNPVYELNDKNWKS  (HPV 16, E1)
GDICNTMHYTNWTHIYICEE  (HPV 16, E2)
FDGDICNTMHYTNWTHIYIC  (HPV 16, E2)
PRSEPDTGNPCHTTKLLHRD  (HPV 16, E2)
TNLDTASTTLLACFLLCFCV  (HPV 16, E5)
FDGNKDNCMTYVAWDSVYYM  (HPV 18, E2)
CCKCEARIELVVESSADDLRAFQQLFLNTL  (HPV 18, E7)
 
QHLPARRAEPQRHTMLCMCCKCEARIELVV (HPV 18, E7)
LCMCCKCEARIELVVESSADDLRAFQQLFL (HPV 18, E7)
ESSADDLRAFQQLFLNTLSFVCPWCASQQ (HPV 18, E7)
QSVAITCQKDAAPAENKDPYD (HPV 18, L1)
CCQCKSTLRLCVQSTQVDIRILQELLMGSF (HPV 31, E7)
YDNDKKNTMDYTNWGEIYII (HPV 33, E2)
CHTCNTTVRLCVNSTASDLRTIQQLLMGTV (HPV 33, E7)
TSQAITCQKTVPPKEKEDPLG (HPV 33, L1)
CCECKFVVQLDIQSTKEDLRVVQQLLMGAL (HPV 56, E7)
 or the detection of a virus specific antigen-antibody complex in the immunoassay being detected by a peptide containing the same epitope or a peptide containing substantial homology with the original peptide.

4. Method for diagnosing the presence of human papillomavirus (HPV) infection and of papillomavirus (PV) carrying tumours by immunoassay, **characterized** in that the detection is performed by immunoassay based on IgM antibodies against any of the peptides 
IGSARMLVKFIDEAQREKC (HPV1, E2)
YDNNPDNQTRHTIWNHVYYQ (HPV 1, E2)
RLGNEHEESNSPLATPCVWP (HPV 6, E4)
FDGCEDNVMEYVVWTHIYLQ (HPV 11, E2)
RRRLGSEHVDRPLTTPCVWP (HPV 11, E4)
QSQAITCQKPTPEKEKQDPYK (HPV 11, L1)
KQHRDAVQVLKRKYLGSCIE (HPV 16, E1)
QYSGGSGGGCSQYSSGSGGE (HPV 16, E1)
SKSHFWLQPLADAKIGMLDD (HPV 16, E1)
DSLPTFKCVSGQNTNTL (HPV 16, E1)
PTGCIKKHGYTVEVQFDGDI (HPV 16, E2)
TYFVQFKDDAEKYSKNKVWEHPV (HPV 16, E2)
PRSEPDTGNPCHTTKLLHRD (HPV 16, E2)
FNSSHKGRINCNSNTTPIVH (HPV 16, E2)
TNLDTASTTLLACFLLCFCV (HPV 16, E5)
YSKISEYRHYCYSLYGTTLE (HPV 16, E6)
GTTLEQQYNKPLCDLLIRCI (HPV 16, E6)
 
FDGNKDNCMTYVAWDSVYYM (HPV 18, E2)
QHLPARRAEPQRHTMLCMCCKCEARIELVV (HPV 18, E7)
LCMCCKCEARIELVVESSADDLRAFQQLFL (HPV 18, E7)
ESSADDLRAFQQLFLNTLSFVCPWCASQQ (HPV 18, E7)
QSVAITCQKDAAPAENKDPYD (HPV 18, L1)
YDNDKKNTMDYTNWGEIYII (HPV 33, E2)
PQTPPSPLQSCSVQTPPWTI (HPV 33, E4)
TSQAITCQKTVPPKEKEDPLG (HPV 33, L1)
 or the detection of a virus specific antigen-antibody complex in the immunoassay being detected by a peptide containing the same epitope or a peptide containing substantial homology with the original peptide.

5. Method as in any of claims 1-4, **characterized** in that the immuoassay is ELISA.

6. Method as in any of claims 1-4, **characterized** in that the immunoassay is performed on serum.

7. Method as in any of claims 1-4, **characterized** in that the immunoassay is performed on cervical secretions.

8. Method as in any of claims 1-4, **characterized** in that the immuoassay measures the presence of cervix cancer, condyloma, CIN, warts or squamous cell carcinoma in the skin.

9. Method as in any of claims 1-4, **characterized** in that the immuoassay is immunofluorescence.

10. Method as in any of claims 1-4, **characterized** in that the immuoassay is immunohistocytochemistry.

11. Method as in any of claims 1-4, **characterized** in that the immunoassay is performed on secretions gathered with a brush or a spatula.

## Patentansprüche

1. Verfahren zum Diagnostizieren des Vorhandenseins von menschlicher Papillomavirus (HPV) -Infektion und von Papillomavirus(PV)-tragenden Tumoren durch Immuntest, dadurch gekennzeichnet, daß der Nachweis durchgeführt wird durch Immuntest auf der Basis von irgendeinem der Peptide 
IGSARMLVKFIDEAQREKC (HPV 1, E2)
YDNNPDNQTRHTIWNHVYYQ (HPV 1, E2)
CAYCEKLVRLTVLADHSAIRQLEELLLRSL (HPV 1, E7)
FDGCANNTMDYVVWTDVYVQ (HPV 6, E2)
RLGNEHEESNSPLATPCVWP (HPV 6, E4)
CCGCDSNVRLVVQCTETDIREVQQLLLGTL (HPV 6, E7)
FDGCEDNVMEYVVWTHIYLQ (HPV 11, E2)
RRRLGSEHVDRPLTTPCVWP (HPV 11, E4)
CCGCDSNVRLVVECTDGDIRQLQDLLLGTL (HPV 11, E7)
QSQAITCQKPTPEKEKQDPYK (HPV 11, L1)
KQHRDAVQVLKRKYLGSCIE (HPV 16, E1)
QYSGGSGGGCSQYSSGSGGE (HPV 16, E1)
GSGGEGVSERHTICQTPLTN (HPV 16, E1)
KAAMLAKFKELYGVSFSELV (HPV 16, E1)
SKSHFWLQPLADAKIGMLDD (HPV 16, E1)
GMLDDATVPCWNYIDDNLRN (HPV 16, E1)
LHNRLVVFTFPNEFPFDENG (HPV 16, E1)
FDENGNPVYELNDKNWKS (HPV 16, E1)
GDICNTMHYTNWTHIYICEE (HPV 16, E2)
PTGCIKKHGYTVEVQFDGDI (HPV 16, E2)
FDGDICNTMHYTNWTHIYIC (HPV 16, E2)
QVDYYGLYYVHEGIRTYFVQ (HPV 16, E2)
TYFVQFKDDAEKYSKNKVWE (HPV 16, E2)
PRSEPDTGNPCHTTKLLHRD (HPV 16, E2)
TNLDTASTTLLACFLLCFCV (HPV 16, E5)
YSKISEYRHYCYSLYGTTLE (HPV 16, E6)
GTTLEQQYNKPLCDLLIRCI (HPV 16, E6)
FDGNKDNCMTYVAWDSVYYM (HPV 18, E2)
SLLNSYSTPPHRIPAPCPWA (HPV 18, E4)
 
CCKCEARIELVVESSADDLRAFQQLFLNTL (HPV 18, E7)
HGPKATLQDIVLHLEPQNEIPVDLLCHEQL (HPV 18, E7)
QHLPARRAEPQRHTMLCMCCKCEARIELVV (HPV 18, E7)
LCMCCKCEARIELVVESSADDLRAFQQLFL (HPV 18, E7)
ESSADDLRAFQQLFLNTLSFVCPWCASQQ (HPV 18, E7)
QSVAITCQKDAAPAENKDPYD (HPV 18, L1)
CCQCKSTLRLCVQSTQVDIRILQELLMGSF (HPV 31, E7)
YDNDKKNTMDYTNWGEIYII (HPV 33, E2)
PQTPPSPLQSCSVQTPPWTI (HPV 33, E4)
CHTCNTTVRLCVNSTASDLRTIQQLLMGTV (HPV 33, E7)
CCECKFVVQLDIQSTKEDLRVVQQLLMGAL (HPV 56, E7)
 oder den Nachweis eines virusspezifischen Antigen-Antikörper-Komplexes im Immuntest, nachgewiesen mit einem Peptid, das dasselbe Epitop enthält, oder einem Peptid, das wesentliche Homologie mit dem ursprünglichen Peptid enthält.

2. Verfahren zum Diagnostizieren des Vorhandenseins von menschlicher Papillomavirus(HPV)-Infektion oder von Papillomavirus(PV)-tragenden Tumoren durch Immuntest, dadurch gekennzeichnet, daß der Nachweis durchgeführt wird durch Immuntest auf der Basis von IgA-Antikörpern gegen irgendeines der Peptide 
CAYCEKLVRLTVLADHSAIRQLEELLLRSL (HPV 1, E7)
FDGCANNTMDYVVWTDVYVQ (HPV 6, E2)
CCGCDSNVRLVVQCTETDIREVQQLLLGTL (HPV 6, E7)
FDGCEDNVMEYVVWTHIYLQ (HPV 11, E2)
CCGCDSNVRLVVECTDGDIRQLQDLLLGTL (HPV 11, E7)
QYSGGSGGCSQYSSGSGGE (HPV 16, E1)
GSGGEGVSERHTICQTPLTN (HPV 16, E1)
KAAMLAKFKELYGVSFSELV (HPV 16, E1)
SKSHFWLQPLADAKIGMLDD (HPV 16, E1)
GMLDDATVPCWNYIDDNLRN (HPV 16, E1)
LHNRLVVFTFPNEFPFDENG (HPV 16, E1)
FDENGNPVYELNDKNWKS (HPV 16, E1)
GDICNTMHYTNWTHIYICEE (HPV 16, E2)
FDGDICNTMHYTNWTHIYIC (HPV 16, E2)
QVDYYGLYYVHEGIRTYFVQ (HPV 16, E2)
 
TYFVQFKDDAEKYSKNKVWE (HPV 16, E2)
PRSEPDTGNPCHTTKLLHRD (HPV 16, E2)
TNLDTASTTLLACFLLCFCV (HPV 16, E5)
FDGNKDNCMTYVAWDSVYYM (HPV 18, E2)
CCKCEARIELVVESSADDLRAFQQLFLNTL (HPV 18, E7)
CCQCKSTLRLCVQSTQVDIRILQELLMGSF (HPV 31, E7)
CHTCNTTVRLCVNSTASDLRTIQQLLMGTV (HPV 33, E7)
CCECKFVVQLDIQSTKEDLRVVQQLLMGAL (HPV 56, E7)
 oder den Nachweis eines virusspezifischen Antigen-Antikörper-Komplexes im Immuntest, nachgewiesen mit einem Peptid, das dasselbe Epitop enthält, oder einem Peptid, das wesentliche Homologie mit dem ursprünglichen Peptid enthält.

3. Verfahren zum Diagnostizieren des Vorhandenseins von menschlicher Papillomavirus(HPV)-Infektion oder von Papillomavirus(PV)-tragenden Tumoren durch Immuntest, dadurch gekennzeichnet, daß der Nachweis durchgeführt wird durch Immuntest auf der Basis von IgG-Antikörpern gegen irgendeines der Peptide 
IGSARMLVKFIDEAQREKC (HPV1, E2)
YDNNPDNQTRHTIWNHVYYQ (HPV 1, E2)
CAYCEKLVRLTVLADHSAIRQLEELLLRSL (HPV 1, E7)
FDGCANNTMDYVVWTDVYVQ (HPV 6, E2)
RLGNEHEESNSPLATPCVWP (HPV 6, E4)
CCGCDSNVRLVVQCTETDIREVQQLLLGTL (HPV 6, E7)
FDGCEDNVMEYVVWTHIYLQ (HPV 11, E2)
CCGCDSNVRLVVECTDGDIRQLQDLLLGTL (HPV 11, E7)
QSQAITCQKPTPEKEKQDPYK (HPV 11, L1)
GMLDDATVPCWNYIDDNLRN (HPV 16, E1)
FDENGNPVYELNDKNWKS (HPV 16, E1)
GDICNTMHYTNWTHIYICEE (HPV 16, E2)
FDGDICNTMHYTNWTHIYIC (HPV 16, E2)
PRSEPDTGNPCHTTKLLHRD (HPV 16, E2)
TNLDTASTTLLACFLLCFCV (HPV 16, E5)
FDGNKDNCMTYVAWDSVYYM (HPV 18, E2)
CCKCEARIELVVESSADDLRAFQQLFLNTL (HPV 18, E7)
 
QHLPARRAEPQRHTMLCMCCKCEARIELVV (HPV 18, E7)
LCMCCKCEARIELVVESSADDLRAFQQLFL (HPV 18, E7)
ESSADDLRAFQQLFLNTLSFVCPWCASQQ (HPV 18, E7)
QSVAITCQKDAAPAENKDPYD (HPV 18, L1)
CCQCKSTLRLCVQSTQVDIRILQELLMGSF (HPV 31, E7)
YDNDKKNTMDYTNWGEIYII (HPV 33, E2)
CHTCNTTVRLCVNSTASDLRTIQQLLMGTV (HPV 33, E7)
TSQAITCQKTVPPKEKEDPLG (HPV 33, L1)
CCECKFVVQLDIQSTKEDLRVVQQLLMGAL (HPV 56, E7)
 oder dem Nachweis eines virusspezifischen Antigen-Antikörper-Komplexes im Immuntest, nachgewiesen mit einem Peptid, das dasselbe Epitop enthält, oder einem Peptid, das wesentliche Homologie mit dem ursprünglichen Peptid enthält.

4. Verfahren zum Diagnostizieren des Vorhandenseins von menschlicher Papillomavirus(HPV)-Infektion oder von Papillomavirus(PV)-tragenden Tumoren durch Immuntest, dadurch gekennzeichnet, daß der Nachweis durchgeführt wird durch Immuntest auf der Basis von IgM-Antikörpern gegen irgendeines der Peptide 
IGSARMLVKFIDEAQREKC (HPV1, E2)
YDNNPDNQTRHTIWNHVYYQ (HPV 1, E2)
RLGNEHEESNSPLATPCVWP (HPV 6, E4)
FDGCEDNVMEYVVWTHIYLQ (HPV 11, E2)
RRRLGSEHVDRPLTTPCVWP (HPV 11, E4)
QSQAITCQKPTPEKEKQDPYK (HPV 11, L1)
KQHRDAVQVLKRKYLGSCIE (HPV 16, E1)
QYSGGSGGGCSQYSSGSGGE (HPV 16, E1)
SKSHFWLQPLADAKIGMLDD (HPV 16, E1)
DSLPTFKCVSGQNTNTL (HPV 16, E1)
PTGCIKKHGYTVEVQFDGDI (HPV 16, E2)
TYFVQFKDDAEKYSKNKVWEHPV (HPV 16, E2)
PRSEPDTGNPCHTTKLLHRD (HPV 16, E2)
FNSSHKGRINCNSNTTPIVH (HPV 16, E2)
TNLDTASTTLLACFLLCFCV (HPV 16, E5)
YSKISEYRHYCYSLYGTTLE (HPV 16, E6)
GTTLEQQYNKPLCDLLIRCI (HPV 16, E6)
 
FDGNKDNCMTYVAWDSVYYM (HPV 18, E2)
QHLPARRAEPQRHTMLCMCCKCEARIELVV (HPV 18, E7)
LCMCCKCEARIELVVESSADDLRAFQQLFL (HPV 18, E7)
ESSADDLRAFQQLFLNTLSFVCPWCASQQ (HPV 18, E7)
QSVAITCQKDAAPAENKDPYD (HPV 18, L1)
YDNDKKNTMDYTNWGEIYII (HPV 33, E2)
PQTPPSPLQSCSVQTPPWTI (HPV 33, E4)
TSQAITCQKTVPPKEKEDPLG (HPV 33, L1)
 oder den Nachweis eines virusspezifischen Antigen-Antikörper-Komplexes im Immuntest, nachgewiesen mit einem Peptid, das dasselbe Epitop enthält, oder einem Peptid, das wesentliche Homologie mit dem ursprünglichen Peptid enthält.

5. Verfahren nach einem der Ansprüche 1 bis 4, dadurch gekennzeichnet, daß der Immuntest ELISA ist.

6. Verfahren nach einem der Ansprüche 1 bis 4, dadurch gekennzeichnet, daß der Immuntest auf Serum durchgeführt wird.

7. Verfahren nach einem der Ansprüche 1 bis 4, dadurch gekennzeichnet, daß der Immuntest auf Cervixsekreten durchgeführt wird.

8. Verfahren nach einem der Ansprüche 1 bis 4, dadurch gekennzeichnet, daß der Immuntest das Vorhandensein von Cervixkrebs, Kondylom, CIN, Warzen oder Plattenepithelkarzinom in der Haut mißt.

9. Verfahren nach einem der Ansprüche 1 bis 4, dadurch gekennzeichnet, daß der Immuntest Immunfluoreszenz ist.

10. Verfahren nach einem der Ansprüche 1 bis 4, dadurch gekennzeichnet, daß der Immuntest Immunhistocytochemie ist.

11. Verfahren nach einem der Ansprüche 1 bis 4, dadurch gekennzeichnet, daß der Immuntest auf Sekreten durchgeführt wird, die mit einer Bürste oder einem Spatel aufgenommen werden.

## Revendications

1. Procédé de diagnostic par des essais immunologiques de la présence d'une infection au papillomavirus humain (HPV) et des tumeurs véhiculant le papillomavirus (PV), caractérisé en ce que la détection est effectuée par des essais immunologiques basés sur l'un quelconque des peptides : 
IGSARMLVKFIDEAQREKC (HPV 1, E2)
YDNNPDNQTRHTIWNHVYYQ (HPV 1, E2)
CAYCEKLVRLTVLADHSAIRQLEELLLRSL (HPV 1, E7)
FDGCANNTMDYVVWTDVYVQ (HPV 6, E2)
RLGNEHEESNSPLATPCVWP (HPV 6, E4)
CCGCDSNVRLVVQCTETDIREVQQLLLGTL (HPV 6, E7)
FDGCEDNVMEYVVWTHIYLQ (HPV 11, E2)
RRRLGSEHVDRPLTTPCVWP (HPV 11, E4)
CCGCDSNVRLVVECTDGDIRQLQDLLLGTL (HPV 11, E7)
QSQAITCQKPTPEKEKQDPYK (HPV 11, L1)
KQHRDAVQVLKRKYLGSCIE (HPV 16, E1)
QYSGGSGGGCSQYSSGSGGE (HPV 16, E1)
GSGGEGVSERHTICQTPLTN (HPV 16, E1)
KAAMLAKFKELYGVSFSELV (HPV 16, E1)
SKSHFWLQPLADAKIGMLDD (HPV 16, E1)
GMLDDATVPCWNYIDDNLRN (HPV 16, E1)
LHNRLVVFTFPNEFPFDENG (HPV 16, E1)
FDENGNPVYELNDKNWKS (HPV 16, E1)
GDICNTMHYTNWTHIYICEE (HPV 16, E2)
PTGCIKKHGYTVEVQFDGDI (HPV 16, E2)
FDGDICNTMHYTNWTHIYIC (HPV 16, E2)
QVDYYGLYYVHEGIRTYFVQ (HPV 16, E2)
TYFVQFKDDAEKYSKNKVWE (HPV 16, E2)
PRSEPDTGNPCHTTKLLHRD (HPV 16, E2)
TNLDTASTTLLACFLLCFCV (HPV 16, E5)
YSKISEYRHYCYSLYGTTLE (HPV 16, E6)
GTTLEQQYNKPLCDLLIRCI (HPV 16, E6)
FDGNKDNCMTYVAWDSVYYM (HPV 18, E2)
SLLNSYSTPPHRIPAPCPWA (HPV 18, E4)
 
CCKCEARIELVVESSADDLRAFQQLFLNTL (HPV 18, E7)
HGPKATLQDIVLHLEPQNEIPVDLLCHEQL (HPV 18, E7)
QHLPARRAEPQRHTMLCMCCKCEARIELVV (HPV 18, E7)
LCMCCKCEARIELVVESSADDLRAFQQLFL (HPV 18, E7)
ESSADDLRAFQQLFLNTLSFVCPWCASQQ (HPV 18, E7)
QSVAITCQKDAAPAENKDPYD (HPV 18, L1)
CCQCKSTLRLCVQSTQVDIRILQELLMGSF (HPV 31, E7)
YDNDKKNTMDYTNWGEIYII (HPV 33, E2)
PQTPPSPLQSCSVQTPPWTI (HPV 33, E4)
CHTCNTTVRLCVNSTASDLRTIQQLLMGTV (HPV 33, E7)
CCECKFVVQLDIQSTKEDLRVVQQLLMGAL (HPV 56, E7)
 ou bien la détection d'un complexe anticorps antigène spécifique du virus dans l'essai immunologique est effectuée par un peptide contenant le même épitope ou un peptide présentant une homologie substantielle avec le peptide original.

2. Procédé de diagnostic par des essais immunologiques de la présence d'une infection au papillomavirus humain (HPV) et des tumeurs véhiculant le papillomavirus (PV), caractérisé en ce que la détection est effectuée par un essai immunologique basé sur des anticorps IgA contre l'un quelconque des peptides : 
CAYCEKLVRLTVLADHSAIRQLEELLLRSL (HPV 1, E7)
FDGCANNTMDYVVWTDVYVQ (HPV 6, E2)
CCGCDSNVRLVVQCTETDIREVQQLLLGTL (HPV 6, E7)
FDGCEDNVMEYVVWTHIYLQ (HPV 11, E2)
CCGCDSNVRLVVECTDGDIRQLQDLLLGTL (HPV 11, E7)
QYSGGSGGGCSQYSSGSGGE (HPV 16, E1)
GSGGEGVSERHTICQTPLTN (HPV 16, E1)
KAAMLAKFKELYGVSFSELV (HPV 16, E1)
SKSHFWLQPLADAKIGMLDD (HPV 16, E1)
GMLDDATVPCWNYIDDNLRN (HPV 16, E1)
LHNRLVVFTFPNEFPFDENG (HPV 16, E1)
FDENGNPVYELNDKNWKS (HPV 16, E1)
GDICNTMHYTNWTHIYICEE (HPV 16, E2)
FDGDICNTMHYTNWTHIYIC (HPV 16, E2)
QVDYYGLYYVHEGIRTYFVQ (HPV 16, E2)
 
TYFVQFKDDAEKYSKNKVWE (HPV 16, E2)
PRSEPDTGNPCHTTKLLHRD (HPV 16, E2)
TNLDTASTTLLACFLLCFCV (HPV 16, E5)
FDGNKDNCMTYVAWDSVYYM (HPV 18, E2)
CCKCEARIELVVESSADDLRAFQQLFLNTL (HPV 18, E7)
CCQCKSTLRLCVQSTQVDIRILQELLMGSF (HPV 31, E7)
CHTCNTTVRLCVNSTASDLRTIQQLLMGTV (HPV 33, E7)
CCECKFVVQLDIQSTKEDLRVVQQLLMGAL (HPV 56, E7)
 ou bien la détection d'un complexe anticorps antigène spécifique du virus dans l'essai Immunologique est effectuée par un peptide contenant le même épitope ou un peptide présentant une homologie substantielle avec le peptide original.

3. Procédé de diagnostic par des essais immnologiques de la présence d'une infection au papillomavirus humain (HPV) et des tumeurs véhiculant le papillomavirus (PV), caractérisé en ce que la détection est effectuée par un essai immunologique basé sur des anticorps IgG contre l'un quelconque des peptides : 
IGSARMLVKFIDEAQREKC (HPV1, E2)
YDNNPDNQTRHTIWNHVYYQ (HPV 1, E2)
CAYCEKLVRLTVLADHSAIRQLEELLLRSL (HPV 1, E7)
FDGCANNTMDYVVWTDVYVQ (HPV 6, E2)
RLGNEHEESNSPLATPCVWP (HPV 6, E4)
CCGCDSNVRLVVQCTETDIREVQQLLLGTL (HPV 6, E7)
FDGCEDNVMEYVVWTHIYLQ (HPV 11, E2)
CCGCDSNVRLVVECTDGDIRQLQDLLLGTL (HPV 11, E7)
QSQAITCQKPTPEKEKQDPYK (HPV 11, L1)
GMLDDATVPCWNYIDDNLRN (HPV 16, E1)
FDENGNPVYELNDKNWKS (HPV 16, E1)
 
GDICNTMHYTNWTHIYICEE (HPV 16, E2)
FDGDICNTMHYTNWTHIYIC (HPV 16, E2)
PRSEPDTGNPCHTTKLLHRD (HPV 16, E2)
TNLDTASTTLLACFLLCFCV (HPV 16, E5)
FDGNKDNCMTYVAWDSVYYM (HPV 18, E2)
CCKCEARIELVVESSADDLRAFQQLFLNTL (HPV 18, E7)
QHLPARRAEPQRHTMLCMCCKCEARIELVV (HPV 18, E7)
LCMCCKCEARIELVVESSADDLRAFQQLFL (HPV 18, E7)
ESSADDLRAFQQLFLNTLSFVCPWCASQQ (HPV 18, E7)
QSVAITCQKDAAPAENKDPYD (HPV 18, L1)
CCQCKSTLRLCVQSTQVDIRILQELLMGSF (HPV 31, E7)
YDNDKKNTMDYTNWGEIYII (HPV 33, E2)
CHTCNTTVRLCVNSTASDLRTIQQLLMGTV (HPV 33, E7)
TSQAITCQKTVPPKEKEDPLG (HPV 33, L1)
CCECKFVVQLDIQSTKEDLRVVQQLLMGAL (HPV 56, E7)
 ou bien la détection d'un complexe anticorps antigène spécifique du virus dans l'essai immunologique est effectuée par un peptide contenant le même épitope ou un peptide présentant une homologie substantielle avec le peptide original.

4. Procédé de diagnostic par des essais immunologiques de la présence d'une infection au papillomavirus humain (HPV) et des tumeurs véhiculant le papillomavirus (PV), caractérisé en ce que la détection est effectuée par un essai immunologique basé sur des anticorps IgM contre l'un quelconque des peptides : 
IGSARMLVKFIDEAQREKC (HPV1, E2)
YDNNPDNQTRHTIWNHVYYQ (HPV 1, E2)
RLGNEHEESNSPLATPCVWP (HPV 6, E4)
FDGCEDNVMEYVVWTHIYLQ (HPV 11, E2)
RRRLGSEHVDRPLTTPCVWP (HPV 11, E4)
QSQAITCQKPTPEKEKQDPYK (HPV 11, L1)
 
KQHRDAVQVLKRKYLGSCIE (HPV 16, E1)
QYSGGSGGCSQYSSGSGGE (HPV 16, E1)
SKSHFWLQPLADAKIGMLDD (HPV 16, E1)
DSLPTFKCVSGQNTNTL (HPV 16, E1)
PTGCIKKHGYTVEVQFDGDI (HPV 16, E2)
TYFVQFKDDAEKYSKNKVWEHPV (HPV 16, E2)
PRSEPDTGNPCHTTKLLHRD (HPV 16, E2)
FNSSHKGRINCNSNTTPIVH (HPV 16, E2)
TNLDTASTTLLACFLLCFCV (HPV 16, E5)
YSKISEYRHYCYSLYGTTLE (HPV 16, E6)
GTTLEQQYNKPLCDLLIRCI (HPV 16, E6)
FDGNKDNCMTYVAWDSVYYM (HPV 18, E2)
QHLPARRAEPQRHTMLCMCCKCEARIELVV (HPV 18, E7)
LCMCCKCEARIELVVESSADDLRAFQQLFL (HPV 18, E7)
ESSADDLRAFQQLFLNTLSFVCPWCASQQ (HPV 18, E7)
QSVAITCQKDAAPAENKDPYD (HPV 18, L1)
YDNDKKNTMDYTNWGEIYII (HPV 33, E2)
PQTPPSPLQSCSVQTPPWTI (HPV 33, E4)
TSQAITCQKTVPPKEKEDPLG (HPV 33, L1)
 ou bien la détection d'un complexe anticorps antigène spécifique du virus dans l'essai immunologique est effectuée par un peptide contenant le même épitope ou un peptide présentant une homologie substantielle avec le peptide original.

5. Procédé selon l'une quelconque des revendications 1 à 4, caractérisé en ce que l'essai immunologique est ELISA.

6. Procédé selon l'une quelconque des revendications 1 à 4, caractérisé en ce que l'essai immunologique est effectué sur du sérum.

7. Procédé selon l'une quelconque des revendications 1 à 4, caractérisé en ce que l'essai immunologique est effectué sur des sécrétions cervicales.

8. Procédé selon l'une quelconque des revendications 1 à 4, caractérisé en ce que l'essai immunologique mesure la présence du cancer du col de l'utérus, le condylome, CIN, le carcimone des verrues ou des cellules squameuses dans la peau.

9. Procédé selon l'une quelconque des revendications 1 à 4, caractérisé en ce que l'essai immunologique est l'immunofuorescence.

10. Procédé selon l'une quelconque des revendications 1 à 4, caractérisé en ce que l'essai immunologique est l'immunohistocytochimie.

11. Procédé selon l'une quelconque des revendications 1 à 4, caractérisé en ce que l'essai immunologique est effectué sur des sécrétions recueillies au moyen d'une brosse ou d'une spatule.
